# EUROPÄISCHE PATENTSCHRIFT

(11) **EP 1 305 292 B1**
(45) Veröffentlichungstag und Bekanntmachung des Hinweises auf die Patenterteilung: **20.06.2012**
(21) Anmeldenummer: 01956525.8
(22) Anmeldetag: 11.07.2001
(51) Int. Cl.: C07D 231/44, C07D 277/56, C07D 207/34, C07D 213/82, C07D 309/30, C07D 307/68, C07D 333/38, C07D 327/06, C07D 263/34, C07C 251/48, C07C 233/65, C07C 211/45, C07C 223/06, A01N 43/00

(54) **BIPHENYLCARBOXAMIDE**
BIPHENYL CARBOXAMIDES
BIPHENYLCARBOXAMIDES

(30) Priorität: 24.07.2000 DE 10035857; 09.05.2001 DE 10122447
(43) Veröffentlichungstag der Anmeldung: 02.05.2003
(73) Patentinhaber: Bayer CropScience AG, 40789 Monheim (DE)
(72) Erfinder: ELBE, Hans-Ludwig, 42329 Wuppertal (DE); RIECK, Heiko, 69110 Ste. Foy-lès-Lyon (FR); DUNKEL, Ralf, 40789 Monheim (DE); WACHENDORFF-NEUMANN, Ulrike, 56566 Neuwied (DE); MAULER-MACHNIK, Astrid, 42799 Leichlingen (DE); KUCK, Karl-Heinz, 40764 Langenfeld (DE); KUGLER, Martin, 42799 Leichlingen (DE); JAETSCH, Thomas, 50670 Köln (DE)
(74) Vertreter: Bader, Axel Jochen
(86) Internationale Anmeldenummer: PCT/EP2001/007981
(87) Internationale Veröffentlichungsnummer: WO 2002/008197

(56) Entgegenhaltungen:
- WO-A-00/09482
- WO-A-00/14071
- WO-A-99/09013
- GB-A- 2 244 486
- US-A- 5 763 450
- HOWARD E. DUNN ET AL: "Arylboronic acids. Imino-derivatives from o-Formylbenzeneboronic acid", JOURNAL OF ORGANIC CHEMISTRY, vol. 33, no. 12, 1968, pages 4483-4486,
- W.H. SCOUTEN ET AL: "Synthesis and X-ray crystal structure investigation of a potential boronate affinity chromatography ligand o-boronobenzalmethoxyamine", JOURNAL OF CHEMICAL CRYSTALLOGRAPHY, vol. 24, no. 9, 1994, pages 621-626, XP009151379,

## Beschreibung

Die vorliegende Erfindung betrifft neue Biphenylcarboxamide, mehrere Verfahren zu deren Herstellung und deren Verwendung zur Bekämpfung von unerwünschten Mikroorganismen.

Es ist bereits bekannt, dass zahlreiche Carboxanilide fungizide Eigenschaften besitzen (vergleiche WO 93/11 117, WO 99/09 013, WO 00/14 071, EP-A 0 545 099 und EP-A 0 589 301). Die Wirksamkeit dieser Stoffe ist gut, lässt aber in manchen Fällen zu wünschen übrig.

Es wurden nun neue Biphenylcarboxamide der Formel (I) 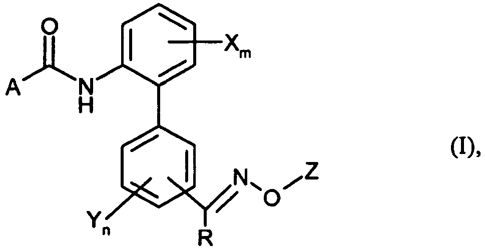 in welcher
- R: für Wasserstoff, C₁-C₆-Alkyl, C₃-C₆-Cycloalkyl oder für C₁-C₆-Halogenalkyl mit 1 bis 5 Halogenatomen steht,
- Z: für Wasserstoff, C₁-C₆-Alkyl oder für C₁-C₆-Halogenalkyl mit 1 bis 5 Halogenatomen steht,
- X und Y: unabhängig voneinander für Halogen, Nitro, Cyano, Hydroxy, Carboxyl, C₁-C₈-Alkyl, C₁-C₆-Halogenalkyl mit 1 bis 5 Halogenatomen, C₁-C₈-Alkoxy, C₁-C₆-Halogenalkoxy mit 1 bis 5 Halogenatomen, C₁-C₈-Alkylthio, C₁-C₆- Halogenalkylthio mit 1 bis 5 Halogenatomen, C₂-C₈-Alkenyloxy, C₂-C₈-Halogenalkenyloxy mit 1 bis 5 Halogenatomen, C₃-C₈-Alkinyloxy, C₃-C₈-Halogenalkinyloxy mit 1 bis 5 Halogenatomen, C₃-C₈-Cycloalkyl, C₁-C₈-Alkoxycarbonyl, C₁-C₈-Alkylsulfinyl, C₁-C₈-Alkylsulfonyl, C₁-C₈-Halogenalkylsulfinyl mit 1 bis 5 Halogenatomen, C₁-C₈-Halogenalkylsulfonyl mit 1 bis 5 Halogenatomen oder C₁-C₆-Alkoximino-C₁-C₆-alkyl stehen,
- m: für ganze Zahlen von 0 bis 3 steht, wobei X für gleiche oder verschiedene Reste steht, wenn m für 2 oder 3 steht,
- n: für ganze Zahlen von 0 bis 4 steht, wobei Y für gleiche oder verschiedene Reste steht, wenn n für 2, 3 oder 4 steht,
und
- A: für einen Rest der Formel 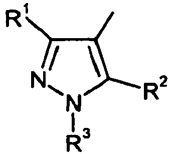 steht, worin
α)
   R¹ für Wasserstoff, Cyano, Halogen, Nitro, C₁-C₄-Alkyl, C₁-C₄-Halogenalkyl mit 1 bis 5 Halogenatomen, C₃-C₆-Cycloalkyl, C₁-C₄-Alkoxy, C₁-C₄-Halogenalkoxy mit 1 bis 5 Halogenatomen, C₁-C₄-Alkylthio, C₁-C₄-Halogenalkylthio mit 1 bis 5 Halogenatomen, Aminocarbonyl, oder Aminocarbonyl-C₁-C₄-alkyl steht und
   R² für Wasserstoff, Cyano, C₁-C₄-Alkyl, C₁-C₄-Alkoxy oder C₁-C₄-Alkylthio steht und
   R³ für Wasserstoff, C₁-C₄-Alkyl, C₁-C₄-Halogenalkyl mit 1 bis 5 Halogenatomen, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Halogenalkylthio-C₁-C₄-alkyl mit 1 bis 5 Halogenatomen, C₁-C₄-Alkoxy-C₁-C₄-alkyl, C₁-C₄-Halogenalkoxy-C₁-C₄-alkyl mit 1 bis 5 Halogenatomen oder Phenyl steht, oder
β)
   R¹ für Wasserstoff, Cyano, Halogen, Nitro, C₁-C₄-Halogenalkyl mit 1 bis 5 Halogenatomen, C₃-C₆-Cycloalkyl, C₁-C₄-Alkoxy, C₁-C₄-Halogenalkoxy mit 1 bis 5 Halogenatomen, C₁-C₄-Alkylthio, C₁-C₄-Halogenalkylthio mit 1 bis 5 Halogenatomen, Aminocarbonyl, oder Aminocarbonyl-C₁-C₄-alkyl steht und
   R² für Fluor steht und
   R³ für Wasserstoff, C₁-C₄-Alkyl, C₁-C₄-Halogenalkyl mit 1 bis 5 Halogenatomen, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Halogenalkylthio-C_{I}-C₄-alkyl mit 1 bis 5 Halogenatomen, C₁-C₄-Alkoxy-C₁-C₄-alkyl, C₁-C₄-Halogenalkoxy-C₁-C₄-alkyl mit 1 bis 5 Halogenatomen oder Phenyl steht, oder
Y)
   R¹ für Wasserstoff, Cyano, Halogen, Nitro, C₁-C₄-Alkyl, C₁-C₄-Halogenalkyl mit 1 bis 5 Halogenatomen, C₃-C₆-Cycloalkyl, C₁-C₄-Alkoxy, C₁-C₄-Halogenalkoxy mit 1 bis 5 Halogenatomen, C₁-C₄-Alkylthio, C₁-C₄-Halogenalkylthio mit 1 bis 5 Halogenatomen, Aminocarbonyl, oder Aminocarbonyl-C₁-C₄-alkyl steht und
   R² für Fluor steht und
   R³ für Wasserstoff, C₁-C₄-Halogenalkyl mit 1 bis 5 Halogenatomen, Hydroxy-C₁-C₄-alkyl, C₂-C₆-Alkenyl, C₃-C₆-Cycloalkyl, C₁-C₄-Alkylthia-C₁-C₄-alkyl, C₁-C₄-Halogenalkylthio-C₁-C₄-alkyl mit 1 bis 5 Halogenatomen, C₁-C₄-Alkoxy-C₁-C₄-alkyl, C₁-C₄-Halogenalkoxy-C₁-C₄-alkyl mit 1 bis 5 Halogenatomen oder Phenyl steht,
gefunden.

Weiterhin wurde gefunden, dass man Biphenylcarboxamide der Formel (I) erhält, indem man
a) Carbonsäure-Derivate der Formel (II) 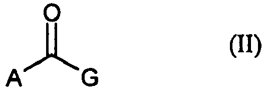 in welcher
   A die oben angegebenen Bedeutungen hat und
   G für Halogen, Hydroxy oder C₁-C₆-Alkoxy steht,
   mit Anilin-Derivaten der Formel (III) 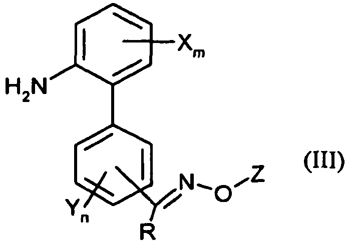 in welcher
      R, Z, X, Y, m und n die oben angegebenen Bedeutungen haben,
      gegebenenfalls in Gegenwart eines Katalysators, gegebenenfalls in Gegenwart eines Säurebindemittels und gegebenenfalls in Gegenwart eines Verdünnungsmittels umsetzt,
   oder
b) Carboxamid-Derivate der Formel (IV) 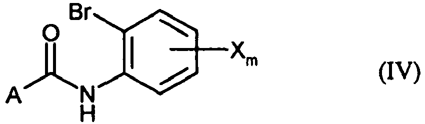 in welcher
   A, X und m die oben angegebenen Bedeutungen haben,
   mit Boronsäure-Derivaten der Formel (V) 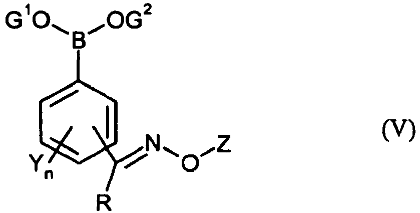 in welcher
      R, Z, Y und n die oben angegebenen Bedeutungen haben und
      G¹ und G² jeweils für Wasserstoff oder zusammen für Tetramethylethylen stehen,
      in Gegenwart eines Katalysators, gegebenenfalls in Gegenwart eines Säurebindemittels und gegebenenfalls in Gegenwart eines Verdünnungsmittels umsetzt,
   oder
c) Carboxamid-Boronsäure-Derivate der Formel (VI) 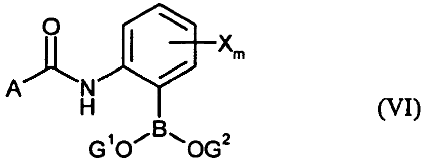 in welcher
   A, X und m die oben angegebenen Bedeutungen haben und
   G¹ und G² jeweils für Wasserstoff oder zusammen für Tetramethylethylen stehen,
   mit Phenyloxim-Derivaten der Formel (VII) 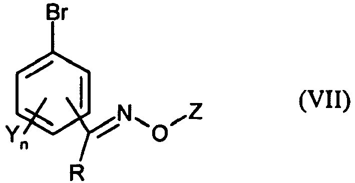 in welcher
      R, Z, Y und n die oben angegebenen Bedeutungen haben,
      in Gegenwart eines Katalysators, gegebenenfalls in Gegenwart eines Säurebindemittels und gegebenenfalls in Gegenwart eines Verdünnungsmittels umsetzt,
   oder
d) Biphenylacyl-Derivate der Formel (VIII) 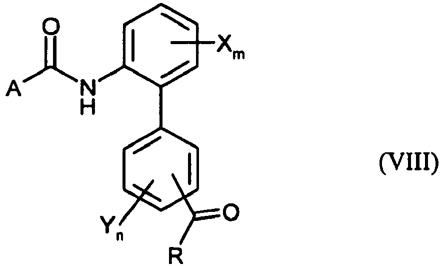 in welcher
   A, R, X, Y, m und n die oben angegebenen Bedeutungen haben,
   mit Alkoxaminen der Formel (IX)

      Z-O-NH₂ x HCl (IX)
   in welcher Z die oben angegebenen Bedeutungen hat,
   gegebenenfalls in Gegenwart eines Säurebindemittels und gegebenenfalls in Gegenwart eines Verdünnungsmittels umsetzt,
   oder
e) Hydroxylamin-Derivate der Formel (I-a) 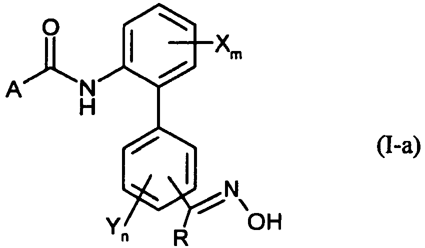 in welcher
   A, R, X, Y, m und n die oben angegebenen Bedeutungen haben,
   mit Verbindungen der Formel (X) 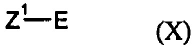 in welcher
      - Z¹: für Cj-C₆-Alkyl steht und
      - E: für Chlor, Brom, Iod, Methansulfonyl oder p-Toluolsulfonyl steht,
      oder
   Z¹ und E zusammen für (Di-C₁-C₆-alkyl)sulfat stehen,
   gegebenenfalls in Gegenwart eines Säurebindemittels und gegebenenfalls in Gegenwart eines Verdünnungsmittels umsetzt,
   oder
f) Carboxamid-Derivate der Formel (IV) 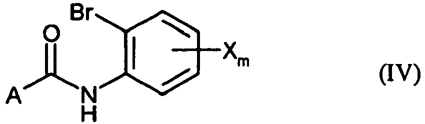 in welcher
   A, X und m die oben angegebenen Bedeutungen haben,
   mit Phenyloxim-Derivaten der Formel (VII) 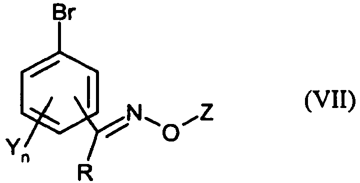 in welcher
      R, Z, Y und n die oben angegebenen Bedeutungen haben,
      in Gegenwart eines Palladium- oder Platin-Katalysators und in Gegenwart von 4,4,4',4',5,5,5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt.

Schließlich wurde gefunden, dass die neuen Biphenylcarboxamide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Biphenylcarboxamide der Formel (I) eine wesentlich bessere fungizide Wirksamkeit als die konstitutionell ähnlichsten, vorbekannten Wirkstoffe gleicher Wirkungsrichtung.

Die erfindungsgemäßen Biphenylcarboxamide sind durch die Formel (I) allgemein definiert.
- R: steht bevorzugt für Wasserstoff, C₁-C₄-Alkyl, C₃-C₆-Cycloalkyl oder C₁-C₄-Halogenalkyl mit 1 bis 5 Halogenatomen.
- Z: steht bevorzugt für Wasserstoff, C₁-C₄-Alkyl oder C₁-C₄-Halogenalkyl mit 1 bis 5 Halogenatomen.
- X und Y: stehen unabhängig voneinander bevorzugt für Fluor, Chlor, Brom, Nitro, Cyano, Hydroxy, Carboxyl, C₁-C₆-Alkyl, C₁-C₂-Halogenalkyl mit 1 bis 5 Fluor-, Chlor- und/oder Bromatomen, C₁-C₆-Alkoxy, C₁-C₂-Halogenalkoxy mit 1 bis 5 Fluor-, Chlor- und/oder Bromatomen, C₁-C₆-Alkylthio, C₁-C₂-Halogenalkylthio mit 1 bis 5 Fluor-, Chlor und/oder Bromatomen, C₂-C₆-Alkenyloxy, C₂-C₆-Halogenalkenyloxy mit 1 bis 5 Halogenatomen, C₃-C₆-Alkinyloxy, C₃-C₆-Halogenalkinyloxy mit 1 bis 5 Halogenatomen, C₃-C₇-Cycloalkyl, C₁-C₄-Alkoxycarbonyl, C₁-C₆-Alkylsulfinyl, C₁-C₆-Alkylsulfonyl, C₁-C₆-Halogenalkylsulfinyl mit 1 bis 5 Halogenatomen, C₁-C₆-Halogenalkylsulfonyl mit 1 bis 5 Halogenatomen oder für C₁-C₄-Alkoximino-C₁-C₄-alkyl.
- m: steht bevorzugt für ganze Zahlen von 0 bis 3, wobei X für gleiche oder verschiedene Reste steht, wenn m für 2 oder 3 steht.
- n: steht bevorzugt für ganze Zahlen von 0 bis 4, wobei Y für gleiche oder verschiedene Reste steht, wenn n für 2, 3 oder 4 steht.
- A: steht bevorzugt für einen Rest der Formel 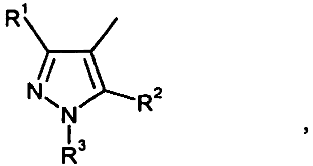 worin
α)
   R¹ für Wasserstoff, Cyano, Fluor, Chlor, Brom, Iod, Methyl, Ethyl, Isopropyl, C₁-C₂-Halogenalkyl mit 1 bis 5 Fluor-, Chlor- und/oder Bromatomen, Cyclopropyl, Methoxy, Ethoxy, C₁-C₂-Halogenalkoxy mit 1 bis 5 Fluor-, Chlor- und/oder Bromatomen, Methylthio, Ethylthio, Trifluormethylthio, Difluormethylthio, Aminocarbonyl, Aminocarbonylinethyl oder Aminocarbonylethyl steht,
   R² für Wasserstoff, Methyl, Ethyl, Methoxy, Ethoxy, Methylthio oder Ethylthio steht und
   R³ für Wasserstoff, Methyl, Ethyl, n-Propyl, Isopropyl, C₁-C₂-Halogenalkyl mit 1 bis 5 Fluor-, Chlor und/oder Bromatomen, Hydroxymethyl, Hydroxyethyl, Cyclopropyl, Cyclopentyl, Cyclohexyl oder Phenyl steht, oder
β)
   R¹ für Wasserstoff, Cyano, Fluor, Chlor, Brom, Iod, C₁-C₂-Halogenalkyl mit 1 bis 5 Fluor-, Chlor- und/oder Bromatomen, Cyclopropyl, Methoxy, Ethoxy, C₁-C₂-Halogenalkoxy mit 1 bis 5 Fluor-, Chlor- und/oder Bromatomen, Methylthio, Ethylthio, Trifluormethylthio, Difluormethylthio, Aminocarbonyl, Aminocarbonylmethyl oder Aminocarbonylethyl steht,
   R² für Fluor steht und
   R³ für Wasserstoff, Methyl, Ethyl, n-Propyl, Isopropyl, C₁-C₂-Halogenalkyl mit 1 bis 5 Fluor-, Chlor und/oder Bromatomen, Hydroxymethyl, Hydroxyethyl, Cyclopropyl, Cyclopentyl, Cyclohexyl oder Phenyl steht, oder
γ)
   R¹ für Wasserstoff, Cyano, Fluor, Chlor, Brom, Iod, Methyl, Ethyl, Isopropyl, C₁-C₂-Halogenalkyl mit 1 bis 5 Fluor-, Chlor- und/oder Bromatomen, Cyclopropyl, Methoxy, Ethoxy, C₁-C₂-Halogenalkoxy mit 1 bis 5 Fluor-, Chlor- und/oder Bromatomen, Methylthio, Ethylthio, Trifluonnethylthio, Difluormethylthio, Aminocarbonyl, Aminocarbonylmethyl oder Aminocarbonylethyl steht,
   R² für Fluor steht und
   R³ für Wasserstoff, , C₁-C₂-Halogenalkyl mit 1 bis 5 Fluor-, Chlor und/oder Bromatomen, Hydroxymethyl, Hydroxyethyl, Cyclopropyl, Cyclopentyl, Cyclohexyl oder Phenyl steht.
- A: steht besonders bevorzugt für einen Rest der Formel 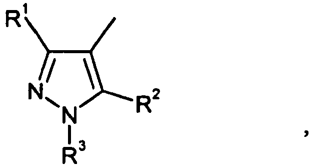 worin
α)
   R¹ für Wasserstoff, Fluor, Chlor, Brom, Iod, Methyl, Ethyl, Isopropyl, Monofluormethyl, Difluormethyl, Trifluormethyl, Difluorchlormethyl, Trichlormethyl, Cyclopropyl, Methoxy, Ethoxy, Trifluormethoxy, Trichlormethoxy, Methylthio, Ethylthio, Trifluormethylthio oder Difluormethylthio steht und
   R² für Wasserstoff, Methyl, Ethyl, Methoxy, Ethoxy, Methylthio oder Ethylthio steht und
   R³ für Wasserstoff, Methyl, Ethyl, Trifluormethyl, Difluormethyl, Hydroxymethyl, Hydroxyethyl oder Phenyl steht, oder
β)
   R¹ für Wasserstoff, Fluor, Chlor, Brom, Iod, Monofluormethyl, Difluormethyl, Trifluormethyl, Difluorchlormethyl, Trichlormethyl, Cyclopropyl, Methoxy, Ethoxy, Trifluormethoxy, Trichlormethoxy, Methylthio, Ethylthio, Trifluormethylthio oder Difluortnethylthio steht und
   R² für Fluor steht und
   R³ für Wasserstoff, Methyl, Ethyl, Trifluormethyl, Difluormethyl, Hydroxymethyl, Hydroxyethyl oder Phenyl steht, oder
γ)
   R¹ für Wasserstoff, Fluor, Chlor, Brom, Iod, Methyl, Ethyl, Isopropyl, Monofluormethyl, Difluormethyl, Trifluormethyl, Difluorchlormethyl, Trichlormethyl, Cyclopropyl, Methoxy, Ethoxy, Trifluormethoxy, Trichlormethoxy, Methylthio, Ethylthio, Trifluormethylthio oder Difluormethylthio steht und
   R² für Fluor steht und
   R³ für Wasserstoff, Trifluormethyl, Difluormethyl, Hydroxymethyl, Hydroxyethyl oder Phenyl steht.

Bevorzugt oder besonders bevorzugt sind Verbindungen, welche die unter bevorzugt oder besonders bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. Mehrere Reste mit denselben Indizes wie beispielsweise m Reste X für m >1, können gleich oder verschieden sein.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können auch einzelne Definitionen entfallen.

Verwendet man 2-Methyl-4-trifluormethyl-1,3-thiazol-5-carbonsäurechlorid und 2-(4-Methoximinomethyl-phenyl)-anilin als Ausgangsstoffe, so kann der Verlauf des erfindungsgemäßen Verfahrens (a) durch das folgende Formelschema veranschaulicht werden.

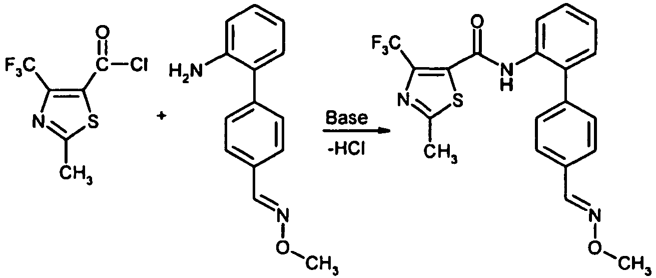

Verwendet man 1,3-Dimethylpyrrol-4-carbonsäure-(2-brom)-anilid und 4-Methoximinoethyl-phenyl-boronsäure als Ausgangsstoffe sowie einen Katalysator, so kann der Verlauf des erfindungsgemäßen Verfahrens (b) durch das folgende Formelschema veranschaulicht werden.

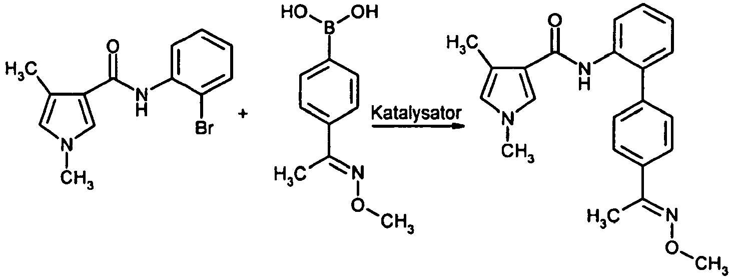

Verwendet man 2-[(1,4-Dimethylpyrrol-3-yl)carbonylamino]phenyl-boronsäure und 1-Brom-4-methoximinoethyl-benzol als Ausgangsstoffe sowie einen Katalysator, so kann der Verlauf des erfindungsgemäßen Verfahrens (c) durch das folgende Formelschema veranschaulicht werden.

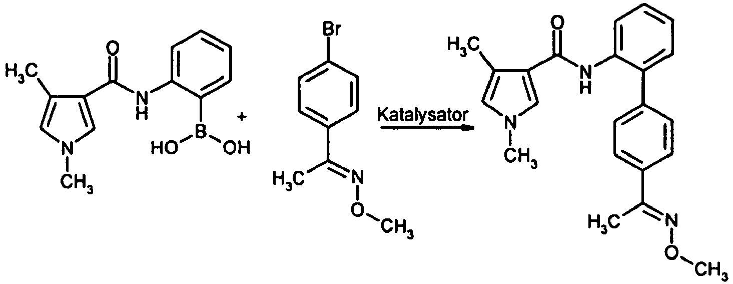

Verwendet man 1-Methyl-3-trifluormethylpyrazol-4-carbonsäure-[2-(4-acetylphenyl)-4-fluor]-anilid und Methoxaminhydrochlorid als Ausgangsstoffe, so kann der Verlauf des erfindungsgemäßen Verfahrens (d) durch das folgende Formelschema veranschaulicht werden.

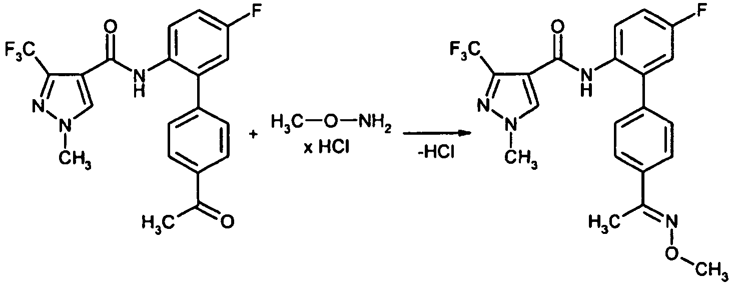

Verwendet man 1,3-Dimethylpyrazol-4-carbonsäure-[2-(4-hydroximinoethyl)-phenyl]-anilid und Methylbromid als Ausgangsstoffe, so kann der Verlauf des erfindungsgemäßen Verfahrens (e) durch das folgende Formelschema veranschaulicht werden.

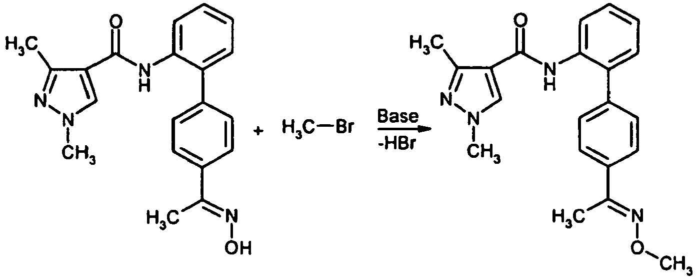

Verwendet man 5-Fluorthiazol-4-carbonsäure-(2-brom)-anilid und 1-Brom-4-methoximinomethyl-benzol als Ausgangsstoffe sowie einen Katalysator und 4,4,4',4-,5,5,5',5'-Octamethyl-2,2'-bis-1,3,2-dioxaborolan, so kann der Verlauf des erfindungsgemäßen Verfahrens (f) durch das folgende Formelschema veranschaulicht werden.

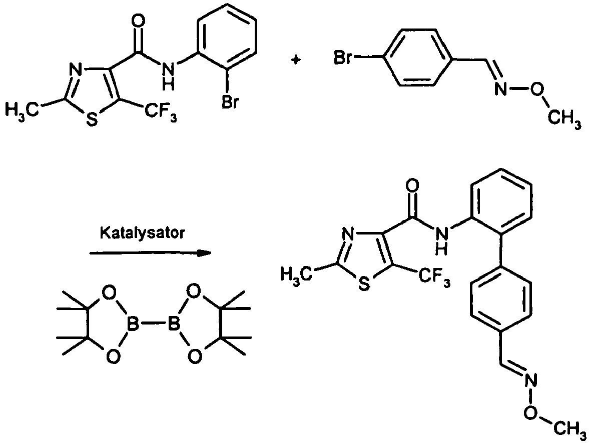

### Erläuterung der Verfahren und Zwischenprodukte

Die bei der Durchführung des erfindungsgemäßen Verfahrens (a) als Ausgangsstoffe benötigten Carbonsäure-Derivate sind durch die Formel (II) allgemein definiert. In dieser Formel steht A vorzugsweise für diejenigen Bedeutungen, die bereits in Zusammenhang mit der Beschreibung der erfindungsgemäßen Stoffe der Formel (I) für diese Reste als bevorzugt bzw. besonders bevorzugt genannt wurden. G steht bevorzugt für Chlor, Brom, Hydroxy, Methoxy oder Ethoxy, besonders bevorzugt für Chlor, Hydroxy oder Methoxy.

Die Carbonsäure-Derivate der Formel (II) sind bekannt oder lassen sich nach bekannten Verfahren herstellen (vgl. WO 93/11 117, EP-A 0 545 099, EP-A 0 589 301 und EP-A 0 589 313).

Die bei der Durchführung des erfindungsgemäßen Verfahrens (a) als Reaktionskomponenten benötigten Anilin-Derivate sind durch die Formel (III) allgemein definiert. In dieser Formel haben R, Z, X, Y, m und n vorzugsweise diejenigen Bedeutungen, die bereits im Zusammenhang mit der Beschreibung der erfindungsgemäßen Stoffe der Formel (I) bevorzugt bzw. besonders bevorzugt für diese Reste bzw. diese Indices genannt wurden.
Die Anilin-Derivate der Formel (III) sind neu. Sie lassen sich teilweise nach bekannten Methoden herstellen (vgl. EP-A 0 545 099 und EP-A 0 589 301).
Man erhält Anilin-Derivate der Formel (III) außerdem, indem man
g) 2-Halogenanilin-Derivate der allgemeinen Formel (XI) 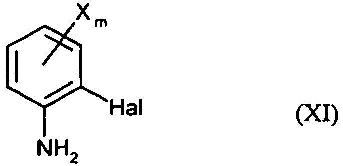 in welcher
   X und m die oben angegebenen Bedeutungen haben und
   Hal für Halogen steht,
   mit Boronsäure-Derivaten der Formel (V) 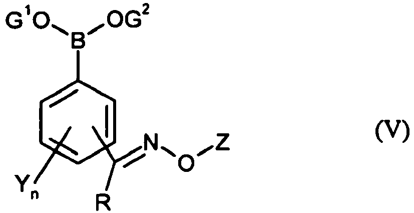 in welcher
      R, Z, Y, n, G¹ und G² die oben angegebenen Bedeutungen haben,
      gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt
   oder
h) Anilinboronsäuren der Formel (XII) 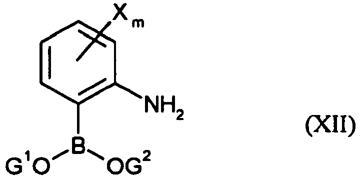 in welcher
   X, m, G¹ und G² die oben angegebenen Bedeutungen haben
   mit Phenyloxim-Derivaten der Formel (VII) 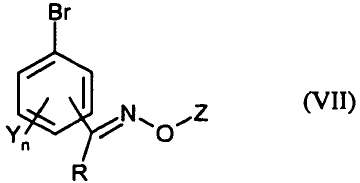 in welcher
      R, Z, Y und n die oben angegebenen Bedeutungen haben,
      gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt.

Die bei der Durchführung des erfindungsgemäßen Verfahrens (g) als Reaktionskomponenten benötigten 2-Halogenanilin-Derivate sind durch die Formel (XI) allgemein definiert. In dieser Formel haben X und m vorzugsweise diejenigen Bedeutungen, die bereits im Zusammenhang mit der Beschreibung der erfindungsgemäßen Stoffe der Formel (I) bevorzugt bzw. besonders bevorzugt für diese Reste bzw. diese Indices genannt wurden. Hal steht vorzugsweise für Fluor, Chlor oder Brom, besonders bevorzugt für Chlor oder Brom.

Die 2-Halogenanilin-Derivate der Formel (XI) sind kommerziell erhältlich oder lassen sich aus den entsprechenden Nitroverbindungen durch Reduktion herstellen.

Die bei der Durchführung des erfindungsgemäßen Verfahrens (h) als Reaktionskomponenten benötigten Anilinboronsäuren sind durch die Formel (XII) allgemein definiert. In dieser Formel haben X und m vorzugsweise diejenigen Bedeutungen, die bereits im Zusammenhang mit der Beschreibung der erfindungsgemäßen Stoffe der Formel (I) bevorzugt bzw. besonders bevorzugt für diese Reste bzw. diese Indices genannt wurden. G¹ und G² stehen bevorzugt jeweils für Wasserstoff oder zusammen für Tetramethylethylen.

Die Anilinboronsäuren der Formel (XII) sind kommerziell erhältlich.

Die bei der Durchführung der erfindungsgemäßen Verfahren (b) und (f) als Ausgangsstoffe benötigten Carboxamid-Derivate sind durch die Formel (IV) allgemein definiert. In dieser Formel stehen A, X und m vorzugsweise für diejenigen Bedeutungen, die bereits in Zusammenhang mit der Beschreibung der erfindungsgemäßen Stoffe der Formel (I) für diese Reste als bevorzugt bzw. besonders bevorzugt genannt wurden.

Die Carboxamid-Derivate der Formel (IV) sind bekannt oder lassen sich nach bekannten Verfahren herstellen (vgl. WO 91/01311, EP-A 0 371 950).

Die bei der Durchführung des erfindungsgemäßen Verfahrens (b) sowie des Verfahrens (g) zur Herstellung der Reaktionskomponenten benötigten Boronsäure-Derivate sind durch die Formel (V) allgemein definiert. In dieser Formel haben R, Z, Y und n vorzugsweise diejenigen Bedeutungen, die bereits im Zusammenhang mit der Beschreibung der erfindungsgemäßen Stoffe der Formel (I) bevorzugt bzw. besonders bevorzugt für diese Reste bzw. diese Indices genannt wurden. G¹ und G² stehen bevorzugt jeweils für Wasserstoff oder zusammen für Tetramethylethylen.

Die Boronsäure-Derivate der Formel (V) lassen sich herstellen, indem man
i) Phenylboronsäuren der Formel (XIII) 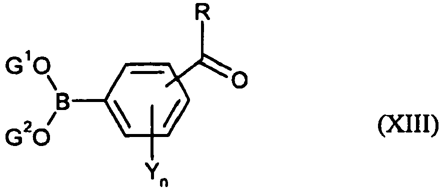 in welcher
   R, Y, n, G¹ und G² die oben angegebenen Bedeutungen haben,
   mit Alkoxaminen der Formel (IX)

      Z-O-NH₂ x HCl (IX)

      in welcher
      Z die oben angegebenen Bedeutungen hat,
      gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt.

Die bei der Durchführung des erfindungsgemäßen Verfahrens (i) als Reaktionskomponenten benötigten Phenylboronsäuren sind durch die Formel (XIII) allgemein definiert. In dieser Formel haben R, Y und n vorzugsweise diejenigen Bedeutungen, die bereits im Zusammenhang mit der Beschreibung der erfindungsgemäßen Stoffe der Formel (I) bevorzugt bzw. besonders bevorzugt für diese Reste bzw. diese Indices genannt wurden. G¹ und G² stehen bevorzugt jeweils für Wasserstoff oder zusammen für Tetramethylethylen.

Die Phenylboronsäuren der Formel (XIII) sind kommerziell erhältlich.

Die bei der Durchführung der erfindungsgemäßen Verfahren (c) als Reaktionskomponenten benötigten Carboxamid-Boronsäure-Derivate sind durch die Formel (VI) allgemein definiert. In dieser Formel stehen A, X und m, vorzugsweise für diejenigen Bedeutungen, die bereits in Zusammenhang mit der Beschreibung der erfindungsgemäßen Stoffe der Formel (I) für diese Reste als bevorzugt bzw. besonders bevorzugt genannt wurden. G¹ und G² stehen bevorzugt jeweils für Wasserstoff oder zusammen für Tetramethylethylen.

Die Carboxamid-Boronsäure-Derivate der Formel (VI) sind neu. Sie lassen sich herstellen, indem man
j) Carbonsäure-Derivate der Formel (II) 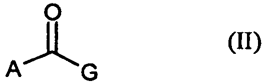 in welcher
   A und G die oben angegebenen Bedeutungen haben,
   mit Anilinboronsäuren der Formel (XII) 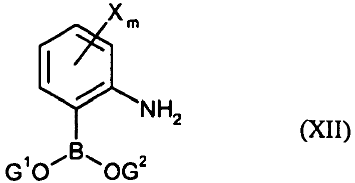 in welcher
      X, m, G¹ und G² die oben angegebenen Bedeutungen haben,
      gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt.

Die bei der Durchführung der erfindungsgemäßen Verfahren (c) und (f) sowie des Verfahrens (h) als Reaktionskomponenten benötigten Phenyloxim-Derivate sind durch die Formel (VII) allgemein definiert. In dieser Formel stehen R, Z, Y und n vorzugsweise für diejenigen Bedeutungen, die bereits in Zusammenhang mit der Beschreibung der erfindungsgemäßen Stoffe der Formel (I) für diese Reste als bevorzugt bzw. besonders bevorzugt genannt wurden.

Die Phenyloxim-Derivate der Formel (VII) sind bekannt oder lassen sich nach bekannten Verfahren herstellen (vgl. Synth. Commun. 2000, 30, 665-669, Synth. Commun. 1999, 29, 1697-1701).

Die bei der Durchführung des erfindungsgemäßen Verfahrens (d) als Ausgangsstoffe benötigten Biphenylacyl-Derivate sind durch die Formel (VIII) allgemein definiert. In dieser Formel stehen A, R, X, Y, m und n für diejenigen Bedeutungen, die bereits in Zusammenhang mit der Beschreibung der erfindungsgemäßen Stoffe der Formel (I) für diese Reste als bevorzugt bzw. besonders bevorzugt genannt wurden.

Die Biphenylacyl-Derivate der Formel (VIII) sind neu. Sie lassen sich herstellen, indem man
k) Carbonsäure-Derivate der Formel (II) 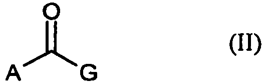 in welcher
   A und G die oben angegebenen Bedeutungen haben,
   mit 2-Benzaldehyd-anilin-Derivaten der Formel (XIV) 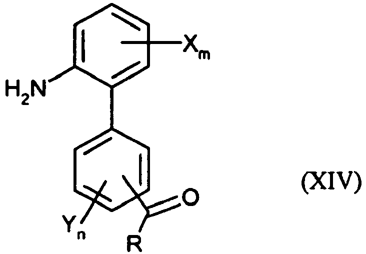 in welcher
      R, X, Y, m und n die oben angegebenen Bedeutungen haben,
      gegebenenfalls in Gegenwart eines Säurebindemittels, sowie gegebenenfalls in Gegenwart eines inerten organischen Verdünnungsmittels umsetzt.

Die bei der Durchführung des erfindungsgemäßen Verfahrens (k) als Reaktionskomponenten benötigten 2-Benzaldehyd-anilin-Derivate sind durch die Formel (XIV) allgemein definiert. In dieser Formel stehen R, X, Y, m und n vorzugsweise für diejenigen Bedeutungen, die bereits in Zusammenhang mit der Beschreibung der erfindungsgemäßen Stoffe der Formel (I) für diese Reste als bevorzugt bzw. besonders bevorzugt genannt wurden.

Die 2-Benzaldehyd-anilin-Derivate der Formel (XIV) lassen sich herstellen, indem man
I) Anilin-Derivate der Formel (XI) 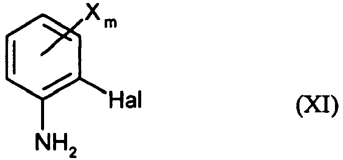 in welcher
   X und m die oben angegebenen Bedeutungen haben und
   Hal für Halogen steht,
   mit Phenylboronsäure-Derivaten der Formel (XIII) 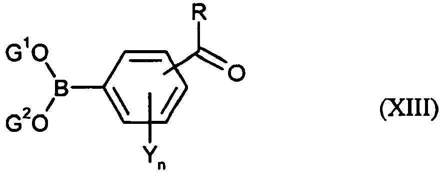 in welcher
      R, Y, n, G¹ und G² die oben angegebenen Bedeutungen haben,
      gegebenenfalls in Gegenwart eines Säurebindemittels, sowie gegebenenfalls in Gegenwart eines inerten organischen Verdünnungsmittels umsetzt.

Die bei der Durchführung des Verfahrens (1) als Reaktionskomponenten benötigten Anilin-Derivate der Formel (XI) wurden bereits bei der Beschreibung des Verfahrens (g) beschrieben.

Die bei der Durchführung des Verfahrens (I) als Reaktionskomponenten benötigten Phenylboronsäure-Derivate der Formel (XIII) wurden bereits bei der Beschreibung des Verfahrens (i) beschrieben.

Die bei der Durchführung des erfindungsgemäßen Verfahrens (d) sowie des Verfahrens (i) als Reaktionskomponenten benötigten Alkoxamine sind durch die Formel (IX) allgemein definiert. In dieser Formel hat Z vorzugsweise diejenigen Bedeutungen, die bereits im Zusammenhang mit der Beschreibung der erfindungsgemäßen Stoffe der Formel (I) bevorzugt bzw. besonders bevorzugt für diesen Rest genannt wurden. Bevorzugt werden die in der Beschreibung angegebenen Hydrochloride eingesetzt. Es können aber auch die freien Alkoxamine in dem erfindungsgemäßen Verfahren verwendet werden.

Die Alkoxamine der Formel (IX) sind kommerziell erhältlich.

Die bei der Durchführung des erfindungsgemäßen Verfahrens (e) als Ausgangsstoffe benötigten Hydroxylamin-Derivate sind durch die Formel (I-a) allgemein definiert. In dieser Formel steht A, R, X, Y, m und n vorzugsweise für diejenigen Bedeutungen, die bereits in Zusammenhang mit der Beschreibung der erfindungsgemäßen Stoffe der Formel (I) für diese Reste als bevorzugt bzw. besonders bevorzugt genannt wurden.

Die erfindungsgemäßen Hydroxylamin-Derivate der Formel (I-a) lassen sich nach einem der oben beschriebenen erfindungsgemäßen Verfahren (a), (b), (c), (d) oder (f) herstellen.

Die bei der Durchführung des erfindungsgemäßen Verfahrens (e) als Reaktionskomponenten benötigten Verbindungen sind durch die Formel (X) allgemein definiert. In dieser Formel steht Z¹ bevorzugt für C₁-C₄-Alkyl, besonders bevorzugt für Methyl oder Ethyl. E steht bevorzugt für Chlor, Brom, Iod, Methansulfonyl oder p-Toluolsulfonyl. E steht besonders bevorzugt für Chlor oder Brom.

Die Verbindungen der Formel (X) sind kommerziell erhältlich.

Als Säurebindemittel kommen bei der Durchführung der erfindungsgemäßen Verfahren (a), (b), (c), (d), (e) und (f) jeweils alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuss einzusetzen, so dass sie gleichzeitig als Säurebindemittel fungiert.

Als Verdünnungsmittel kommen bei der Durchführung der erfindungsgemäßen Verfahren (a), (b), (c), (d), (e) und (f) jeweils alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Sulfone, wie Sulfolan.

Die Reaktionstemperaturen können bei der Durchführung der erfindungsgemäßen Verfahren (a), (b), (c), (d), (e) und (f) jeweils in einem größeren Bereich variiert werden. Im allgemeinen arbeitet man bei Temperaturen zwischen 0°C und 140°C, vorzugsweise zwischen 10°C und 120°C.

Bei der Durchführung der erfindungsgemäßen Verfahren (a), (b), (c), (d), (e) und (f) arbeitet man im allgemeinen jeweils unter Atmosphärendruck. Es ist aber auch möglich, jeweils unter erhöhtem oder vermindertem Druck zu arbeiten.

Bei der Durchführung des erfindungsgemäßen Verfahrens (a) setzt man auf 1 Mol an Säurehalogenid der Formel (II) im allgemeinen 1 Mol oder auch einen Überschuss an Anilin-Derivat der Formel (III) sowie 1 bis 3 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, die organische Phase abtrennt und nach dem Trocknen unter vermindertem Druck einengt. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (b) setzt man auf 1 Mol an Carboxamid der Formel (IV) im allgemeinen 1 Mol oder auch einen Überschuss an Boronsäure-Derivat der Formel (V) sowie 1 bis 5 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (c) setzt man auf 1 Mol an Carboxamid-Boronsäure-Derivat der Formel (VI) im allgemeinen 1 Mol oder auch einen Überschuss an Phenyloxim-Derivat der Formel (VII) sowie 1 bis 10 Mol an Säurebindemittel und 0.5 bis 5 Molprozent eines Katalysators ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (d) setzt man auf 1 Mol an Biphenylacyl-Derivat der Formel (VIII) im allgemeinen 1 Mol oder auch einen Überschuss an Alkoxamin der Formel (IX) sowie 1 bis 5 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt, mit Wasser und Diisopropylether wäscht und anschließend trocknet. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (e) setzt man auf 1 Mol an Hydroxylamin-Derivat der Formel (I-a) im allgemeinen 1 Mol oder auch einen Überschuss an Reagenz der Formel (X) sowie 1 bis 5 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (f) setzt man auf 1 Mol an Carboxamid-Derivat der Formel (IV) im allgemeinen 1 Mol oder auch einen Überschuss an Phenyloxim-Derivat der Formel (VII) sowie 1 bis 5 Mol an Säurebindemittel ein, sowie 1 bis 5 Mol eines Katalysators. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Altemaria brassicae;
Pseudocercosporella-Arten, wie beispielsweise Pseudocercosporella herpotrichoides.

Die gute Pflanzenvemäglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau einsetzen, wie beispielsweise gegen Venturia-, Botrytis-, Selerotinia-, Rhizoctonia-, Uncinula-, Sphaerotheca-, Podosphaera-, Altemaria- und Colletotrichum-Arten. Mit gutem Erfolg werden auch Reiskrankheiten, wie Pyricularia- und Pellicularia-Arten, bekämpft.

Die erfindungsgemäßen Wirkstoffe eignen sich auch zur Steigerung des Emteertrages. Sie sind außerdem mindertoxisch und weisen eine gute Pflanzenverträglichkeit auf.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgerührt werden. Zu den Pflanzenteilen gehört auch Erntegut sowie vegetatives und generatives Vermehrungsmaterial, beispielsweise Stecklinge, Knollen, Rhizome, Ableger und Samen.
Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan; Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin, Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB), Quinoxyfen,
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyly)-β-(2-phenoxyethylyl-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester,
1-(2,4-Dichlorphenyl)-2-(1H-1,2,4-triazol-1-yl)-ethanon-O-phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethylfsulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluomethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-ß-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methy)-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl-oxy]-2,5-thiophendicarboxylat,
cis-1-(4-Chlorphenyl)-2-(11H-1,2,4-triazol-1-yl)-cycloheptanol,
cis-4-[3-[4-(1,1-Dimethylpropyl)phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin-Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam. Kupfersulfat und andere Kupfer-Zubereitungen.

### Insektizide / Akarizide / Nematizide:

Abamectin, Acephat, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaphorthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Elfusilanate, Emamectin, Empenthrin, Endosulfan, Entomopfthora spp., Esfenvalerate, Ethiofencarb, Ethion, Ethoprophos, Etofenprox, Etoxazole, Etrimphos,
Fenamiphos, Fenazaquin, Fenbutatinoxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren,
Halofenozide, HCH, Heptenophos, Hexaflumuron, Hexythiazox, Hydroprene,
Imidacloprid, Isazophos, Isofenphos, Isoxathion, Ivermectin,
Kempolyederviren,
Lamda-cyhalothrin, Lufenuron,
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron,
Omethoat, Oxamyl, Oxydemethon M,
Paecilomyces fumosoroseus, Parathion A, Parathion M, Permethrin, Phenthoat, Phorat, Phosalon, Phosmet, Phosphamidon, Phoxim, Pirimicarb, Pirimiphos A, Pirimiphos M, Profenophos, Promecarb, Propoxur, Prothiophos, Prothoat, Pymetrozine, Pyraclofos, Pyresmethrin, Pyrethrum, Pyridaben, Pyridathion, Pyrimidifen, Pyriproxifen,
Quinalphos,
Ribavirin,
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-Cypermethrin, Zolaprofos

(1R-cis)-[5-(Phenylmethyl)-3-furanyl]-methyl-3-[(dihydro-2-oxo-3(2H)-furanyliden)-methyl]-2,2-dimethylcyclopropancarboxylat,
(3-Phenoxyphenyl)-methyl-2,2,3,3-tetramethylcyclopropanecarboxylat,
1-[(2-Chlor-5-thiazolyl)methyl]tetrahydro-3,5-dimethyl-N-nitro-1,3,5-triazin-2(1H)-imin,
2-(2-Chlor-6-fluorphenyl)-4-[4-(1,1-diemthylethyl)phenyl]-4,5-dihydro-oxazol, 2-(Acetlyoxy)-3-docecyl- 1,4-naphthalinidion,
2-Chlor-N-[[[4-(1-phenylethoxy)-phenyl]-amino]-carbonyl]-benzamid, 2-Chlor-N-[[[4-(2,2-dichlor-1,1-difluorethoxy)-phenyl]-amino]-carbonyl]-benzmid, 3-Methylphenyl-propylcarbamat,
4-[4-(4-Ethoxyphenyl)-4-methylpentyl]-1-fluor-2-phenoxy-benzol,
4-Chlor-2-(1,1-dimethylethyl-5-[[2-(2,6-dimethyl-4-phenoxyphenoxy)ethyl]thio]-3(2H)-pyridazinon,
4-Chlor-2-(2-chlor-2-methylpropyl]-5-[(6-iod-3-pyridinyl)methoxy]-3(2H)-pyridazinon,
4-Chlor-5[(6-chlor-3-pyridinyl)methoxy]-2-(3,4-dichlorphenyl)-3(2H)-pyridazinon, Bacillus thuringiensis strain EG-2348,
Benzoesäure (2-benzoyl-1-(1,1-dimethylethyl)-hydrazid,
Butan 2,2-dimethyl-3-(2,4-dichlorphenyl)-2-oxo-1-oxaspiro[4.5]dec-3-en-4-yl-ester,
[3-[(6-Chlor-3-pyridinyl)methyl)-2-thiazolidinyliden]-cyanamid,
Dihydro-2-(nitromethylen)-2H-1,3-thiazine-3(4H)-carboxaldehyd,
Ethyl-[2-[[1,6-dihydro-6-oxo-1-(phenylmethyl)-4-pyridazinyl]oxy]ethyl]-carbamat, N-(3,4,4-Trifluor-1-oxo-3-butenyl)-glycin,
N-(4-Chlorphenyl)-3-[4-Difluormethoxy)phenyl]-4,5-dihydro-4-phenyl-1H-pyrazol-1-carboxamid,
N-[(2-Chlor-5-thiazolyl)methyl]-N'-methyl-N"-nitro-guanidin,
N-Methyl-N'-(1-methyl-2-propenyl)-1,2-hydrazindicarbothioamid,
N-Methyl-N'-2-propenyl-1,2-hydrazindicarbothioamid,
O,O-Diethyl-[2-(dipropylamino)-2-oxoethyl]-ethylphosphoramidothioat.

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze,

z.B. gegen Candida-Spezies wie Candida albicans, Candida glabrata, Epidermophyton-Spezies wie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii.

Die Aufzählung dieser Pilze stellt keinesfalls eine Beschränkung des erfaßbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10 000 g/ha, vorzugsweise zwischen 10 und 1000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10 000 g/ha, vorzugsweise zwischen 1 und 5 000 g/ha.

Die zum Schutz technischer Materialien verwendeten Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.%, vorzugsweise von 0,05 bis 1,0 Gew.% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung der erfindungsgemäßen Wirkstoffe geht aus den folgenden Beispielen hervor.

### Herstellungsbeispiele

### Beispiel 1

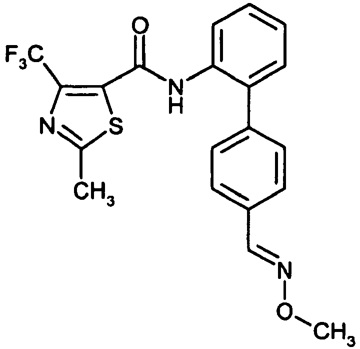

### Verfahren (a):

Eine Lösung von 0,59 g (0,0026 Mol) 2-(4-Methoximinomethyl-phenyl)-anilin in 25 mL Toluol wird bei Raumtemperatur mit 0,26 g (0,0026 Mol) Triethylamin versetzt. In dieses Gemisch lässt man bei Raumtemperatur unter Rühren eine Lösung von 0,6 g (0,0026 Mol) 2-Methyl-4-trifluormethylthiazol-5-carbonsäurechlorid in 5 mL Toluol einlaufen. Nach beendeter Zugabe wird das Reaktionsgemisch auf 50°C erwärmt und 2 h bei dieser Temperatur weiter gerührt. Zur Aufarbeitung wird das Reaktionsgemisch auf Raumtemperatur abgekühlt und mit Wasser versetzt. Die organische Phase wird abgetrennt, über Natriumsulfat getrocknet, filtriert und unter vermindertem Druck eingeengt. Der verbleibende Rückstand wird mit Cyclohexan : Essigsäureethylester = 3:1 als Laufmittel an Kieselgel chromatographiert. Nach dem Einengen des Eluates erhält man 0,81 g (74% der Theorie) an 2-Methyl-4-trifluormethylthiazol-5-carbonsäure-[2-(4-methoximinomethyl-phenyl)]-anilid als Festsubstanz vom Schmelzpunkt 122 bis 123°C.

### Herstellung von Ausgangssubstanzen

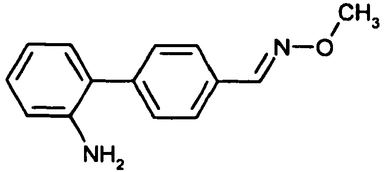

Ein Gemisch aus 2,9 g (0,017 Mol) 2-Bromanilin, 0,68 g Tetrakis-(triphenylphosphin) palladium, 5,5 g (0,031 Mol) 4-Methoximinomethyl-phenyl-boronsäure und 40 mL 1,2-Dimethoxyethan wird bei Raumtemperatur mit einer Lösung von 8,2 g (0,077 Mol) Natriumcarbonat in 35 mL Wasser versetzt. Das Reaktionsgemisch wird anschließend auf Rückfluss-Temperatur gebracht und für 12 h gekocht. Zur Aufarbeitung wird auf Raumtemperatur abgekühlt und mit Diethylether extrahiert. Die organische Phase wird abgetrennt und mit Wasser versetzt. Die organische Phase wird erneut abgetrennt, über Natriumsulfat getrocknet und schließlich unter vermindertem Druck eingeengt. Der verbleibende Rückstand wird mit Cyclohexan : Essigsäureethylester = 3:1 als Laufmittel an Kieselgel chromatographiert. Nach dem Einengen des Eluates erhält man 3,8 g (98,8% der Theorie bezogen auf 2-Bromanilin) an 2-(4-Methoximinomethyl-phenyl)-anilin in Form eines Öles.
¹H-NMR-Spektrum (DMSO/TMS): δ = 3,90 (3H) ppm.

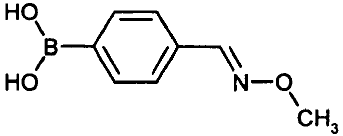

Ein Gemisch aus 5,0 g (0,033 Mol) 4-Formylphenylboronsäure, 3,4 g (0,041 Mol) Methoxylamin-hydrochlorid, 3,4 g (0,041 Mol) Natriumacetat, 40 mL Methanol und 10 mL Wasser wird 12 h bei Raumtemperatur gerührt. Zur Aufarbeitung wird das Reaktionsgemisch in Wasser verrührt, der entstehende Niederschlag abgesaugt, mit Wasser gewaschen und bei 50°C im Vakuum getrocknet. Man erhält 5,56 g (93,1% der Theorie) an 4-Methoximinomethyl-phenyl-boronsäure als farblose Kristalle mit einem Schmelzpunkt von 199 bis 200°C.

Nach den zuvor beschriebenen Methoden werden auch die in der folgenden Tabelle aufgeführten Biphenylcarboxamide der Formel (I) hergestellt.

**Tabelle 1**

| | | |
|---|---|---|
| 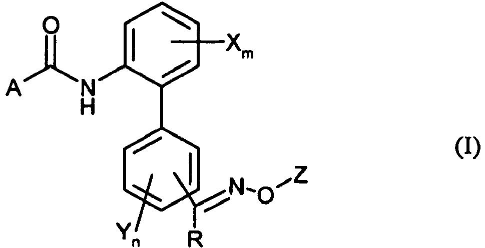 | | |

| **Bsp.-Nr.** | | **Physikal. Konstante** |
|---|---|---|
| 10 | 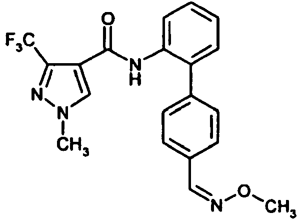 | Fp. 158-160°C |
| 11 | 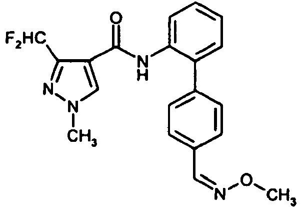 | Fp. 127-129°C |
| 12 | 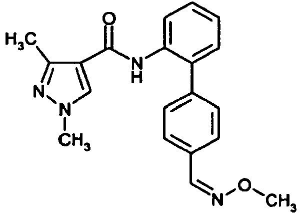 | Fp. 146°C |
| 13 | 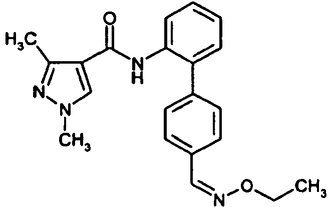 | Fp. 137-139°C |
| 14 | 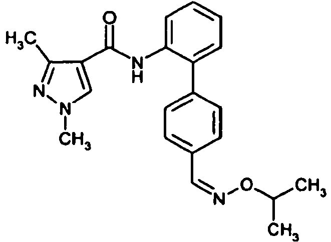 | Fp. 152-153°C |
| 15 | 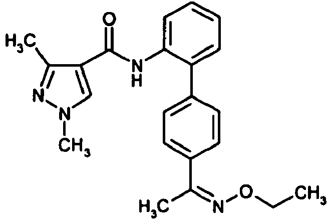 | |
| 16 | 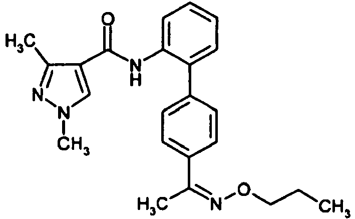 | |
| 17 | 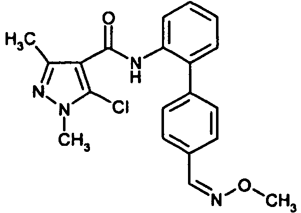 | logP 3,24^{a)} |
| 22 | 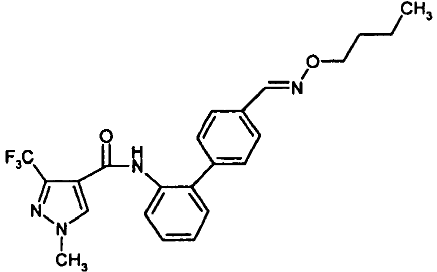 | logP 4,26^{a)} |
| 24 | 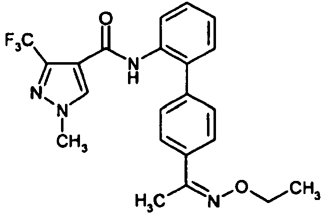 | Fp. 160-162°C |
| 25 | 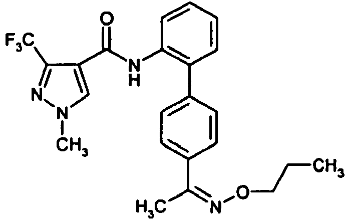 | Fp. 148-150°C |
| 26 | 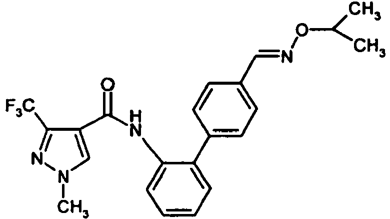 | Fp. 126-128°C |
| 27 | 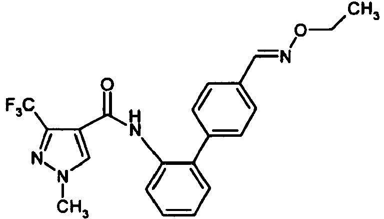 | Fp. 170-172°C |
| 28 | 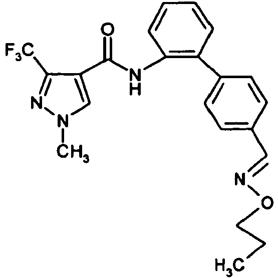 | logP 3,86^{a)} |
| 29 | 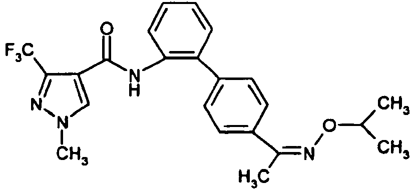 | Fp. 164-166°C |
| 31 | 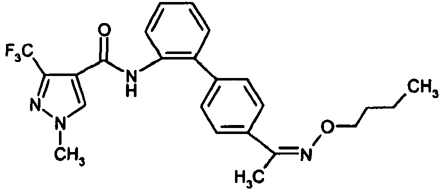 | Fp. 89-91°C |
| 41 | 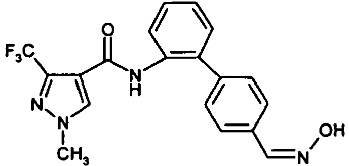 | logP 3,19^{a)} |
| 49 | 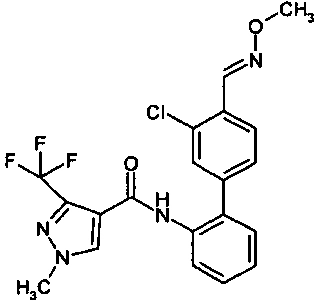 | logP 3,54^{a)} |
| 50 | 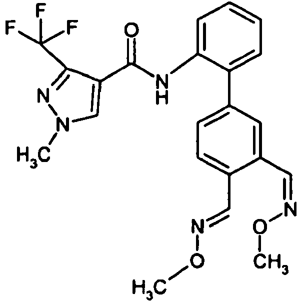 | logP 3,36^{a)} |
| 52 | 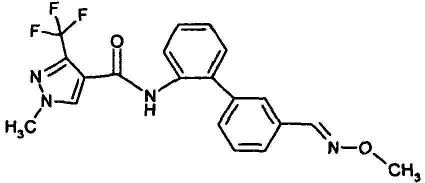 | logP 3,25^{a)} |
| 58 | 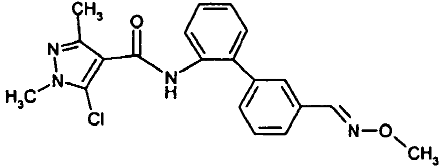 | logP 3,37^{a)} |
| 76 | 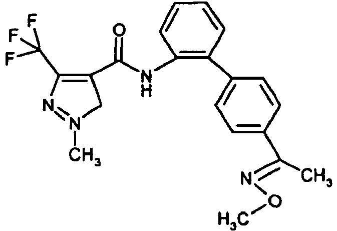 | Fp. 157-158°C |
| 79 | 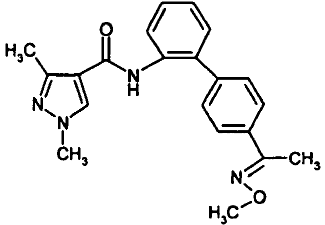 | Fp. 107-109°C |
| 80 | 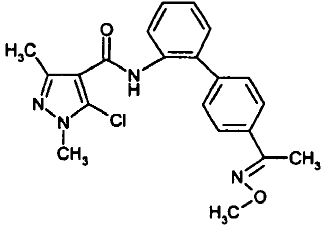 | Fp. 168-171°C |
| 87 | 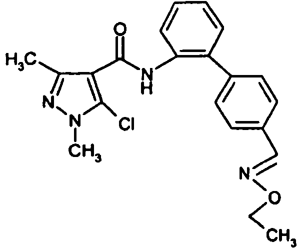 | Fp. 98°C |
| 90 | 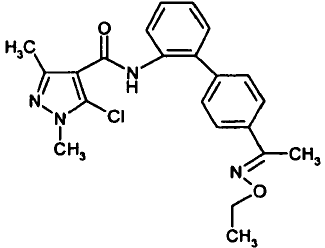 | Fp. 80-82°C |
| 92 | 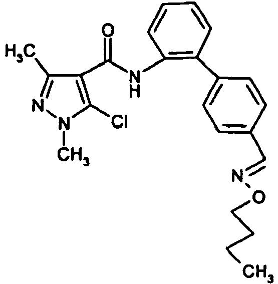 | Fp. 55-57°C |
| 95 | 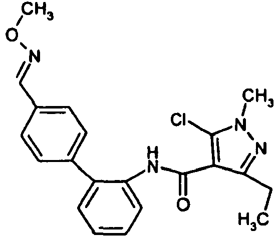 | logP 3,68^{a)} |
| 96 | 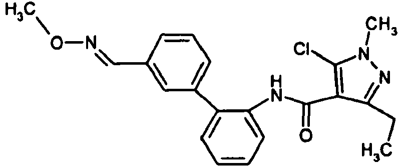 | logP 3,72^{a)} |
| 97 | 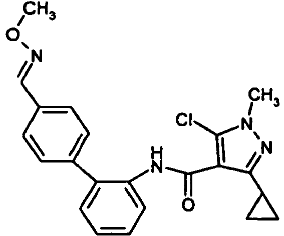 | logP 3,76^{a)} |
| 98 | 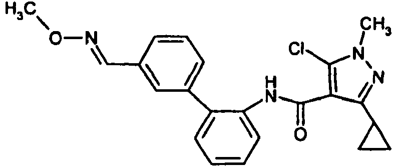 | logP 3,81^{a)} |
| 103 | 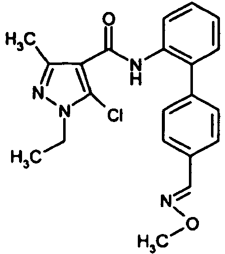 | logP 3,87^{a)} |
| 106 | 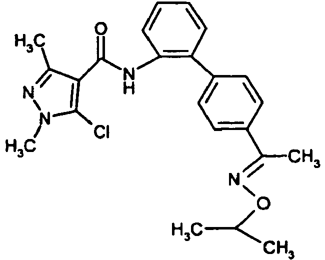 | Fp. 108-109°C |
| 112 | 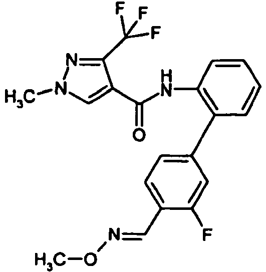 | logP 3,21^{a)} |
| 114 | 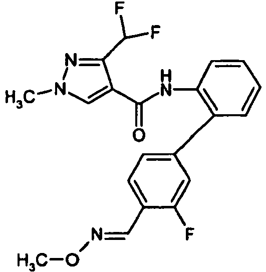 | logP 3,00^{a)} |
| 117 | 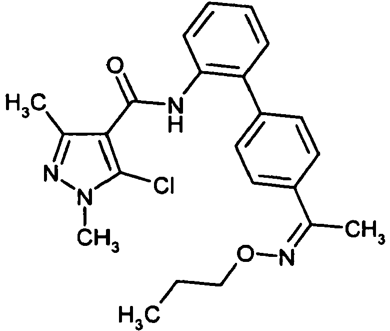 | Fp. 75°C |
| 118 | 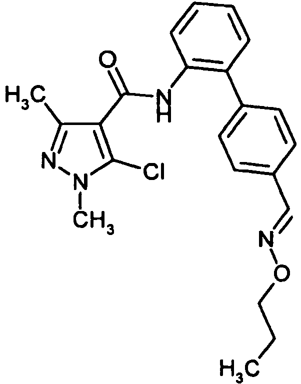 | logP 4.22^{a)} |
| 120 | 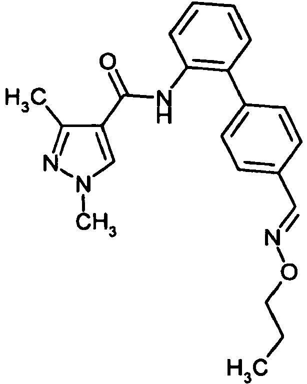 | Fp. 141-143°C |

Die Bestimmung der in Tabelle 1 angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1 % wässrige Phosphorsäure, Acetonitril; linearer Gradient von 10 % Acetonitril bis 90 % Acetonitril - entsprechende Messergebnisse sind in Tabelle 1 mit ^{a)} markiert.
(b) Eluenten für die Bestimmung im neutralen Bereich: 0,01-molare wässrige Phosphatpuffer-Lösung, Acetonitril; linearer Gradient von 10 % Acetonitril bis 90 % Acetonitril - entsprechende Messergebnisse sind in Tabelle 1 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Podosphaera-Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die folgenden erfindungsgemäßen Verbindungen der Herstellungsbeispiele überlegene Wirksamkeit gegenüber dem Stand der Technik:

**Tabelle A: Podosphaera-Test (Apfel) / protektiv**

| Wirkstoff | | Aufwandmenge an Wirkstoff in g/ha | % Wirkungsgrad |
|---|---|---|---|
| (I-10) | 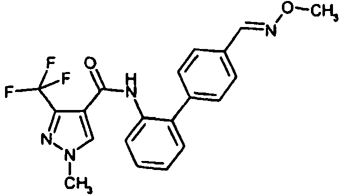 | 100 | 88 |
| (I-11) | 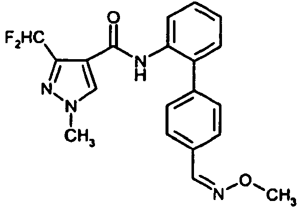 | 100 | 93 |
| (I-22) | 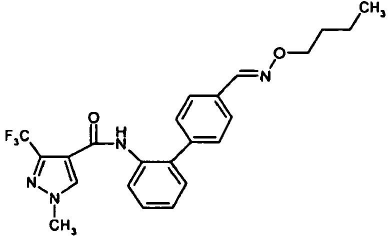 | 100 | 100 |
| (I-24) | 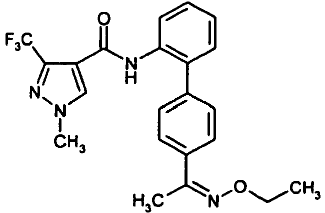 | 100 | 75 |
| (I-26) | 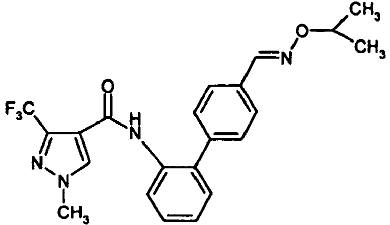 | 100 | 100 |
| (I-28) | 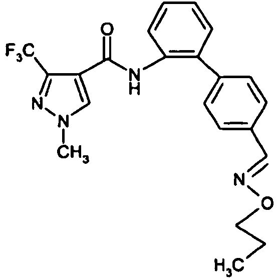 | 100 | 99 |
| (I-41) | 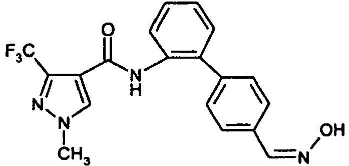 | 100 | 98 |
| (1-49) | 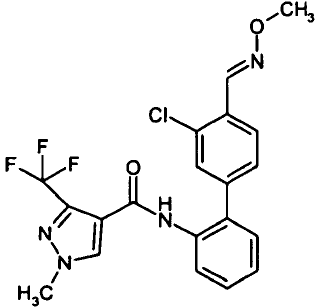 | 100 | 100 |
| (I-50) | 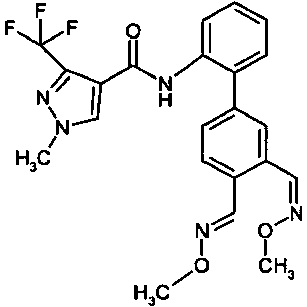 | 100 | 93 |
| (I-52) | 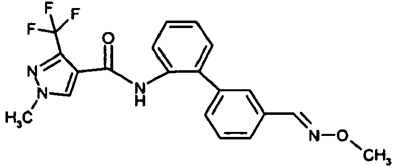 | 100 | 77 |

### Beispiel B

### Sphaerotheca-Test (Gurke) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alk yl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Sphaerotheca fuliginea inokuliert. Die Pflanzen werden dann bei ca. 23°C und einer relativen Luftfeuchtigkeit von ca. 70 % im Gewächshaus aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die folgenden erfindungsgemäßen Verbindungen der Herstellungsbeispiele überlegene Wirksamkeit gegenüber dem Stand der Technik:

**Tabelle B: Sphaerotheca-Test (Gurke) / protektiv**

| Wirkstoff | | Aufwandmenge an Wirkstoff in g/ha | % Wirkungsgrad |
|---|---|---|---|
| (I-1) | 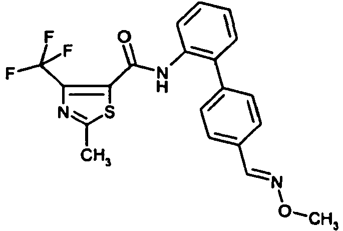 | 100 | 100 |
| (I-3) | 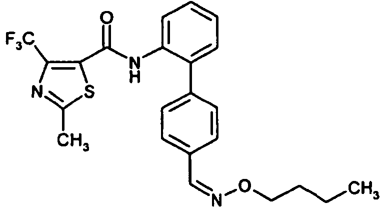 | 100 | 100 |
| (I-4) | 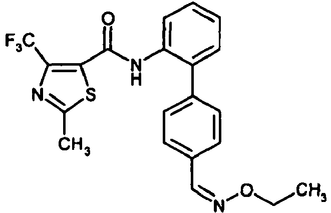 | 100 | 93 |
| (I-5) | 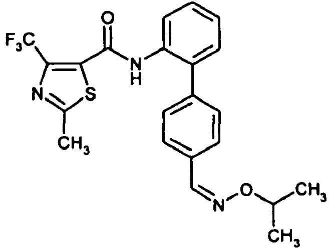 | 100 | 100 |
| (I-10) | 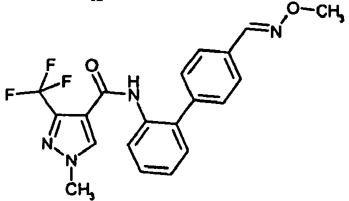 | 100 | 88 |
| (I-11) | 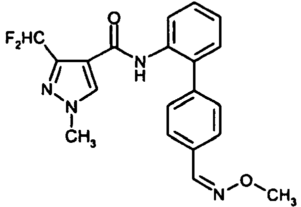 | 100 | 95 |
| (I-22) | 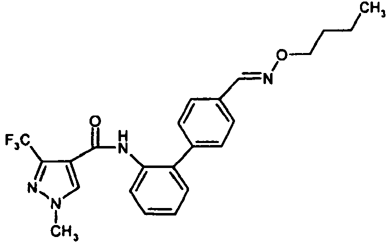 | 100 | 100 |
| (I-24) | 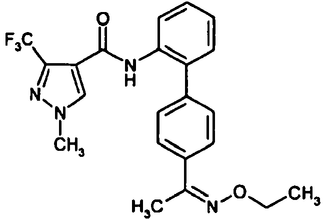 | 100 | 92 |
| (I-26) | 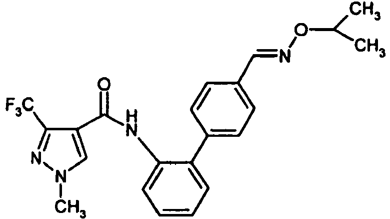 | 100 | 100 |
| (I-28) | 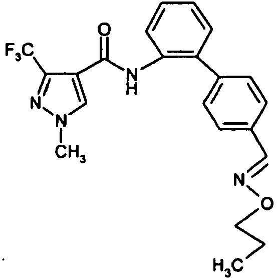 | 100 | 100 |
| (I-41) | 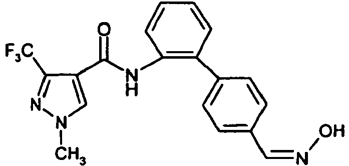 | 100 | 72 |
| (I-49) | 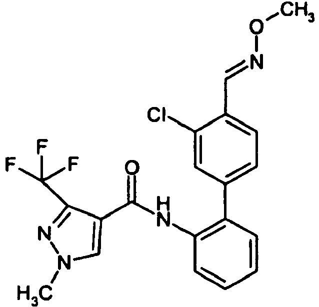 | 100 | 100 |
| (I-50) | 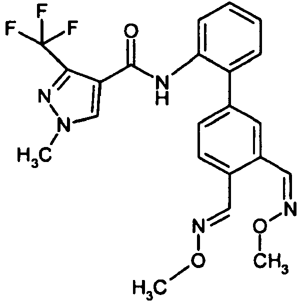 | 100 | 97 |
| (I-52) | 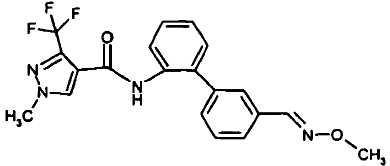 | 100 | 97 |

### Beispiel C

### Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubations-kabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die folgenden erfindungsgemäßen Verbindungen der Herstellungsbeispiele überlegene Wirksamkeit gegenüber dem Stand der Technik:

**Tabelle C: Venturia - Test (Apfel) / protektiv**

| Wirkstoff | | Aufwandmenge an Wirkstoff in g/ha | % Wirkungsgrad |
|---|---|---|---|
| (I-10) | 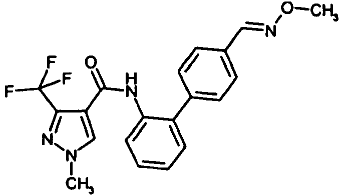 | 100 | 88 |
| (I-11) | 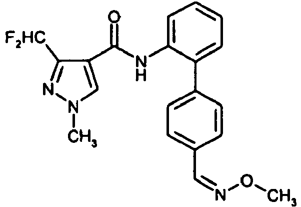 | 100 | 100 |
| (I-22) | 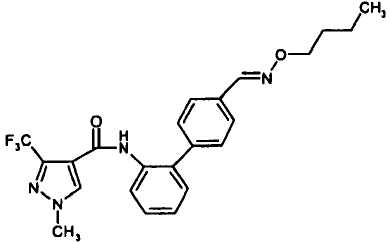 | 100 | 100 |
| (I-24) | 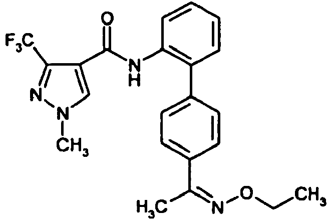 | 100 | 100 |
| (I-26) | 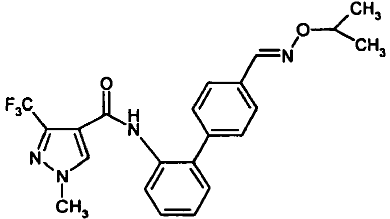 | 100 | 100 |
| (I-28) | 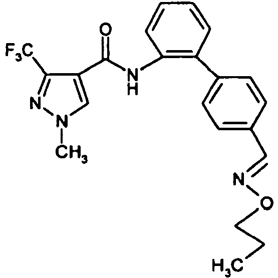 | 100 | 100 |
| (I-41) | 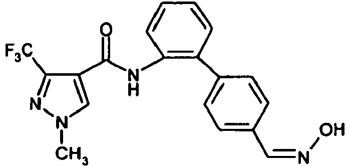 | 100 | 100 |
| (I-49) | 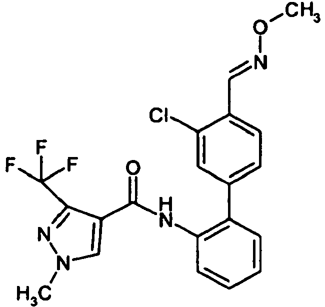 | 100 | 100 |
| (I-50) | 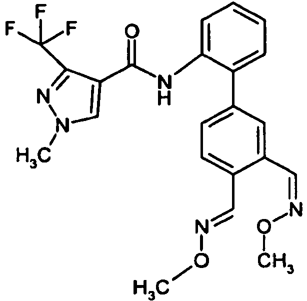 | 100 | 100 |
| (I-52) | 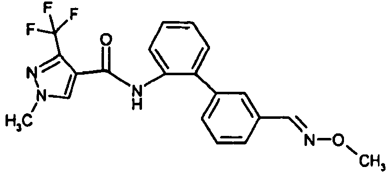 | 100 | 100 |

### Beispiel D

### Puccinia-Test (Weizen) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die folgenden erfindungsgemäßen Verbindungen der Herstellungsbeispiele überlegene Wirksamkeit gegenüber dem Stand der Technik:

**Tabelle D: Puccinia-Test (Weizen) / protektiv**

| Wirkstoff | | Aufwandmenge an Wirkstoff in g/ha | % Wirkungsgrad |
|---|---|---|---|
| (I-10) | 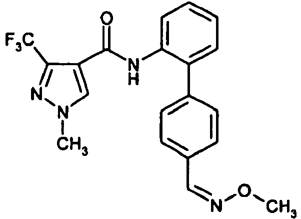 | 250 | 100 |
| (I-49) | 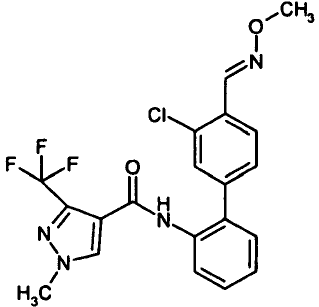 | 250 | 100 |

### Beispiel E

### Alternaria-Test (Tomate) / protektiv

| | |
|---|---|
| Lösungsmittel: | 49 Gewichtsteile N,N-Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von Altemaria solani inokuliert und stehen dann 24 h bei 100 % rel. Feuchte und 20°C. Anschließend stehen die Pflanzen bei 96 % rel. Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die folgenden erfindungsgemäßen Verbindungen der Herstellungsbeispiele überlegene Wirksamkeit gegenüber dem Stand der Technik:

**Tabelle E: Alternaria-Test (Tomate) / protektiv**

| Wirkstoff | | Aufwandmenge an Wirkstoff in g/ha | % Wirkungsgrad |
|---|---|---|---|
| (I-11) | 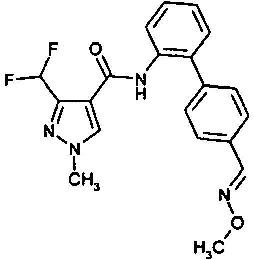 | 750 | 95 |

### Beispiel F

### Hemmtest an Riesenkolonien von Basidiomyceten

Aus Kolonien von Gloeophyllum trabeum, Coniophora puteana, Poria placenta, Lentinus tigrinus und Coriolus versicolor wurden Myzelstücke ausgestochen und auf einem Malzextrakt-Pepton-haltigen Agamährboden bei 26°C inkubiert Die Hemmung des Hyphenwachstums auf wirkstoffhaltigen Nährböden wurde mit dem Längenwachstum auf Nährboden ohne Wirkstoffzusatz verglichen und als prozentuale Hemmung bonitiert.

Bei diesem Test zeigen z.B. die folgenden erfindungsgemäßen Verbindungen der Herstellungsbeispiele gute Wirksamkeit:

**Tabelle F: Hemmtest an Riesenkolonien von Basidiomyceten**

| Wirkstoff | | Aufwandmenge an Wirkstoff in ppm | % Wirkungsgrad |
|---|---|---|---|
| (I-10) | 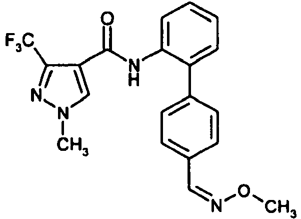 | 6 | 100 |
| (I-11) | 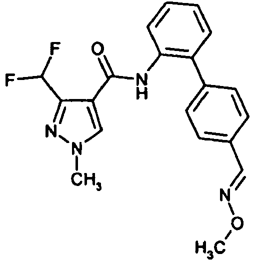 | 6 | 100 |
| (I-12) | 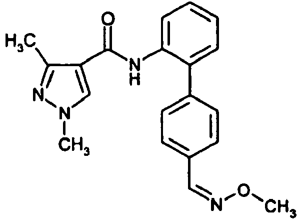 | 6 | 100 |

## Patentansprüche

1. Biphenylcarboxamide der Formel (I) 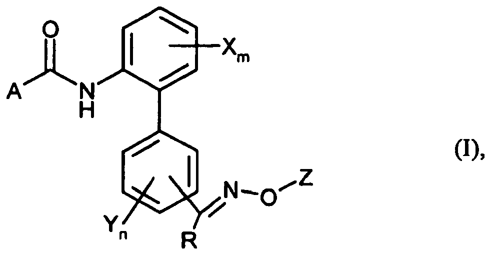 in welcher
R für Wasserstoff, C₁-C₆-Alkyl, C₃-C₆-Cycloalkyl oder für C₁-C₆-Halogenalkyl mit 1 bis 5 Halogenatomen steht,
Z für Wasserstoff, C₁-C₆-Alkyl oder für C₁-C₆-Halogenalkyl mit 1 bis 5 Halogenatomen steht,
X und Y unabhängig voneinander für Halogen, Nitro, Cyano, Hydroxy, Carboxyl, C₁-C₈-Alkyl, C₁-C₆-Halogenalkyl mit 1 bis 5 Halogenatomen, C₁-C₈-Alkoxy, C₁-C₆-Halogenalkoxy mit 1 bis 5 Halogenatomen, C₁-C₈-Alkylthio, C₁-C₆-Halogenalkylthio mit 1 bis 5 Halogenatomen, C₂-C₈-Alkenyloxy, C₂-C₈-Halogenalkenyloxy mit 1 bis 5 Halogenatomen, C₃-C₈-Alkinyloxy, C₃-C₈-Halogenalkinyloxy mit 1 bis 5 Halogenatomen, C₃-C₈-Cycloalkyl, C₁-C₈-Alkoxycarbonyl, C₁-C₈-Alkylsulfinyl, C₁-C₈-Alkylsulfonyl, C₁-C₈-Halogenalkylsulfinyl mit 1 bis 5 Halogenatomen, C₁-C₈-Halogenalkylsulfonyl mit 1 bis 5 Halogenatomen oder C₁-C₆-Alkoximino-C₁-C₆-alkyl stehen,
m für ganze Zahlen von 0 bis 3 steht, wobei X für gleiche oder verschiedene Reste steht, wenn m für 2 oder 3 steht,
n für ganze Zahlen von 0 bis 4 steht, wobei Y für gleiche oder verschiedene Reste steht, wenn n für 2, 3 oder 4 steht,
und
A für einen Rest der Formel 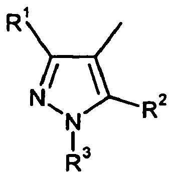 steht, worin
α)
R¹ für Wasserstoff, Cyano, Halogen, Nitro, C₁-C₄-Alkyl, C₁-C₄-Halogenalkyl mit 1 bis 5 Halogenatomen, C₃-C₆-Cycloalkyl, C₁-C₄-Alkoxy, C₁-C₄-Halogenalkoxy mit 1 bis 5 Halogenatomen, C₁-C₄-Alkylthio, C₁-C₄-Halogenalkylthio mit 1 bis 5 Halogenatomen, Aminocarbonyl, oder Aminocarbonyl-C₁-C₄-alkyl steht und
R² für Wasserstoff, Cyano, C₁-C₄-Alkyl, C₁-C₄-Alkoxy oder C₁-C₄-Alkylthio steht und
R³ für Wasserstoff, C₁-C₄-Alkyl, C₁-C₄-Halogenalkyl mit 1 bis 5 Halogenatomen, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Halogenalkylthio-C₁-C₄-alkyl mit 1 bis 5 Halogenatomen, C₁-C₄-Alkoxy-C₁-C₄-alkyl, C₁-C₄-Halogenalkoxy-C₁-C₄-alkyl mit 1 bis 5 Halogenatomen oder Phenyl steht,
oder
β)
R¹ für Wasserstoff, Cyano, Halogen, Nitro, C₁-C₄-Halogenalkyl mit 1 bis 5 Halogenatomen, C₃-C₆- Cycloalkyl, C₁-C₄-Alkoxy, C₁-C₄-Halogenalkoxy mit 1 bis 5 Halogenatomen, C₁-C₄-Alkylthio, C₁-C₄-Halogenalkylthio mit 1 bis 5 Halogenatomen, Aminocarbonyl, oder Aminocarbonyl-C₁-C₄-alkyl steht und
R² für Fluor steht und
R³ für Wasserstoff, C₁-C₄-Alkyl, C₁-C₄-Halogenalkyl mit 1 bis 5 Halogenatomen, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Halogenalkylthio-C₁-C₄-alkyl mit 1 bis 5 Halogenatomen, C₁-C₄-Alkoxy-C₁-C₄-alkyl, C₁-C₄-Halogenalkoxy-C₁-C₄-alkyl mit 1 bis 5 Halogenatomen oder Phenyl steht,
oder
γ)
R¹ für Wasserstoff, Cyano, Halogen, Nitro, C₁-C₄-Alkyl, C₁-C₄-Halogenalkyl mit 1 bis 5 Halogenatomen, C₃-C₆-Cycloalkyl, C₁-C₄-Alkoxy, C₁-C₄-Halogenalkoxy mit 1 bis 5 Halogenatomen, C₁-C₄-Alkylthio, C₁-C₄-Halogenalkylthio mit 1 bis 5 Halogenatomen, Aminocarbonyl, oder Aminocarbonyl-C₁-C₄-alkyl steht und
R² für Fluor steht und
R³ für Wasserstoff, C₁-C₄-Halogenalkyl mit 1 bis 5 Halogenatomen, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Halogenalkylthio-C₁-C₄-alkyl mit 1 bis 5 Halogenatomen, C₁-C₄-Alkoxy-C₁-C₄-alkyl, C₁-C₄-Halogenalkoxy-C₁-C₄-alkyl mit 1 bis 5 Halogenatomen oder Phenyl steht.

2. Biphenylcarboxamide der Formel (I) gemäß Anspruch 1, in welcher
R für Wasserstoff, C₁-C₄-Alkyl, C₃-C₆-Cycloalkyl oder C₁-C₄-Halogenalkyl mit 1 bis 5 Halogenatomen steht,
Z für Wasserstoff, C₁-C₄-Alkyl oder C₁-C₄-Halogenalkyl mit 1 bis 5 Halogenatomen steht,
X und Y unabhängig voneinander für Fluor, Chlor, Brom, Nitro, Cyano, Hydroxy, Carboxyl, C₁-C₆-Alkyl, C₁-C₂-Halogenalkyl mit 1 bis 5 Fluor-, Chlor- und/oder Bromatomen, C₁-C₆-Alkoxy, C₁-C₂-Halogenalkoxy mit 1 bis 5 Fluor-, Chlor- und/oder Bromatomen, C₁-C₆-Alkylthio, C₁-C₂-Halogenalkylthio mit 1 bis 5 Fluor-, Chlor und/oder Bromatomen, C₂-C₆-Alkenyloxy, C₂-C₆-Halogenalkenyloxy mit 1 bis 5 Halogenatomen, C₃-C₆-Alkinyloxy, C₃-C₆-Halogenalkinyloxy mit 1 bis 5 Halogenatomen, C₃-C₇-Cycloalkyl, C₁-C₄-Alkoxycarbonyl, C₁-C₆-Alkylsulfinyl, C₁-C₆-Alkylsulfonyl, C₁-C₆-Halogenalkylsulfinyl mit 1 bis 5 Halogenatomen, C₁-C₆-Halogenalkylsulfonyl mit 1 bis 5 Halogenatomen oder für C₁-C₄-Alkoximino-C₁-C₄-alkyl stehen,
m für ganze Zahlen von 0 bis 3 steht, wobei X für gleiche oder verschiedene Reste steht, wenn m für 2 oder 3 steht,
n für ganze Zahlen von 0 bis 4 steht, wobei Y für gleiche oder verschiedene Reste steht, wenn n für 2, 3 oder 4 steht, und
A für einen Rest der Formel 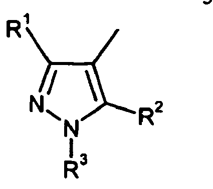 steht, worin
α)
R¹ für Wasserstoff, Cyano, Fluor, Chlor, Brom, Iod, Methyl, Ethyl, Isopropyl, C₁-C₂-Halogenalkyl mit 1 bis 5 Fluor-, Chlor- und/oder Bromatomen, Cyclopropyl, Methoxy, Ethoxy, C₁-C₂-Halogenalkoxy mit 1 bis 5 Fluor-, Chlor- und/oder Bromatomen, Methylthio, Ethylthio, Trifluormethylthio, Difluormethylthio, Aminocarbonyl, Aminocarbonylmethyl oder Aminocarbonylethyl steht,
R² für Wasserstoff, Methyl, Ethyl, Methoxy, Ethoxy, Methylthio oder Ethylthio steht und
R³ für Wasserstoff, Methyl, Ethyl, n-Propyl, Isopropyl, C₁-C₂-Halogenalkyl mit 1 bis 5 Fluor-, Chlor und/oder Bromatomen, Hydroxymethyl, Hydroxyethyl, Cyclopropyl, Cyclopentyl, Cyclohexyl oder Phenyl steht,
oder
β)
R¹ für Wasserstoff, Cyano, Fluor, Chlor, Brom, Iod, C₁-C₂-Halogenalkyl mit 1 bis 5 Fluor-, Chlor- und/oder Bromatomen, Cyclopropyl, Methoxy, Ethoxy, C₁-C₂-Halogenalkoxy mit 1 bis 5 Fluor-, Chlor- und/oder Bromatomen, Methylthio, Ethylthio, Trifluormethylthio, Difluormethylthio, Aminocarbonyl, Aminocarbonylmethyl oder Aminocarbonylethyl steht,
R² für Fluor steht und
R³ für Wasserstoff, Methyl, Ethyl, n-Propyl, Isopropyl, C₁-C₂-Halogenalkyl mit 1 bis 5 Fluor-, Chlor und/oder Bromatomen, Hydroxymethyl, Hydroxyethyl, Cyclopropyl, Cyclopentyl, Cyclohexyl oder Phenyl steht,
oder
γ)
R¹ für Wasserstoff, Cyano, Fluor, Chlor, Brom, Iod, Methyl, Ethyl, Isopropyl, C₁-C₂-Halogenalkyl mit 1 bis 5 Fluor-, Chlor- und/oder Bromatomen, Cyclopropyl, Methoxy, Ethoxy, C₁-C₂-Halogenalkoxy mit 1 bis 5 Fluor-, Chlor- und/oder Bromatomen, Methylthio, Ethylthio, Trifluormethylthio, Difluormethylthio, Aminocarbonyl, Aminocarbonylmethyl oder Aminocarbonylethyl steht,
R² für Fluor steht und
R³ für Wasserstoff, C₁-C₂-Halogenalkyl mit 1 bis 5 Fluor-, Chlor und/oder Bromatomen, Hydroxymethyl, Hydroxyethyl, Cyclopropyl, Cyclopentyl, Cyclohexyl oder Phenyl steht

3. Biphenylcarboxamide der Formel (I) gemäß Anspruch 1, in welcher
R für Wasserstoff, C₁-C₄-Alkyl steht,
Z für Wasserstoff, C₁-C₄-Alkyl steht,
X und Y unabhängig voneinander für Fluor, Chlor, Brom, Nitro, Cyano, Hydroxy, Carboxyl, Methyl, Ethyl, n-Propyl, Isopropyl, n-Butyl, sek.-Butyl, i-Butyl, tert.-Butyl, Trichlormethyl, Trifluormethyl, Difluormethyl, Difluorchlormethyl, Methoxy, Ethoxy, Difluormethoxy, Trifluormethoxy, Methylthio, Trifluormethylthio, Difluorchlormethylthio, Allyloxy, Propargyloxy, Cyclopropyl, Cyclopentyl, Cyclohexyl, Cycloheptyl, Methoxycarbonyl, Ethoxycarbonyl, Methoximinomethyl, Ethoximinomethyl, Methoximinoethyl oder Ethoximinoethyl stehen,
m für ganze Zahlen von 0 bis 3 steht, wobei X für gleiche oder verschiedene Reste steht, wenn m für 2 oder 3 steht,
n für die Zahlen 0 bis 4 steht, wobei Y für gleiche oder verschiedene Reste steht, wenn n für 2, 3 oder 4 steht,
und
A für einen Rest der Formel 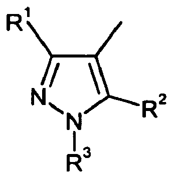 steht, worin
α)
R¹ für Wasserstoff, Fluor, Chlor, Brom, Iod, Methyl, Ethyl, Isopropyl, Monofluormethyl, Difluormethyl, Trifluormethyl, Difluorchlormethyl, Trichlormethyl, Cyclopropyl, Methoxy, Ethoxy, Trifluormethoxy, Trichlormethoxy, Methylthio, Ethylthio, Trifluormethylthio oder Difluormethylthio steht und
R² für Wasserstoff, Methyl, Ethyl, Methoxy, Ethoxy, Methylthio oder Ethylthio steht und
R³ für Wasserstoff, Methyl, Ethyl, Trifluormethyl, Difluormethyl, Hydroxymethyl, Hydroxyethyl oder Phenyl steh,
oder
β)
R¹ für Wasserstoff, Fluor, Chlor, Brom, Iod, Monofluormethyl, Difluormethyl, Trifluormethyl, Difluorchlormethyl, Trichlormethyl, Cyclopropyl, Methoxy, Ethoxy, Trifluormethoxy, Trichlormethoxy, Methylthio, Ethylthio, Trifluormethylthio oder Difluormethylthio steht und
R² für Fluor steht und
R³ für Wasserstoff, Methyl, Ethyl, Trifluormethyl, Difluormethyl, Hydroxymethyl, Hydroxyethyl oder Phenyl steht,
oder
γ)
R¹ für Wasserstoff, Fluor, Chlor, Brom, Iod, Methyl, Ethyl, Isopropyl, Monofluormethyl, Difluormethyl, Trifluormethyl, Difluorchlormethyl, Trichlormethyl, Cyclopropyl, Methoxy, Ethoxy, Trifluormethoxy, Trichlormethoxy, Methylthio, Ethylthio, Trifluormethylthio oder Difluormethylthio steht und
R² für Fluor steht und
R³ für Wasserstoff, Trifluormethyl, Difluormethyl, Hydroxymethyl, Hydroxyethyl oder Phenyl steht

4. Verfahren zur Herstellung von Biphenylcarboxamiden der Formel (I) gemäß Anspruch 1, **dadurch gekennzeichnet, dass** man
a) Carbonsäure-Derivate der Formel (II) 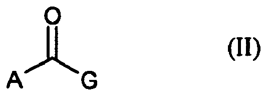 in welcher
A die in Anspruch 1 angegebenen Bedeutungen hat und
G für Halogen, Hydroxy oder C₁-C₆-Alkoxy steht,
mit Anilin-Derivaten der Formel (III) 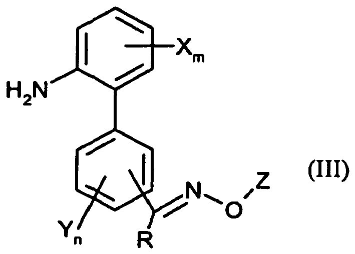 in welcher
R, Z, X, Y, m und n die in Anspruch 1 angegebenen Bedeutungen haben,
gegebenenfalls in Gegenwart eines Katalysators, gegebenenfalls in Gegenwart eines Säurebindemittels und gegebenenfalls in Gegenwart eines Verdünnungsmittels umsetzt,
oder
b) Carboxamid-Derivate der Formel (IV) 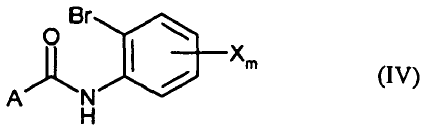 in welcher
A, X und m die in Anspruch 1 angegebenen Bedeutungen haben,
mit Boronsäure-Derivaten der Formel (V) 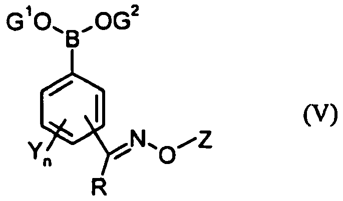 in welcher
R, Z, Y und n die in Anspruch 1 angegebenen Bedeutungen haben und
G¹ und G² jeweils für Wasserstoff oder zusammen für Tetramethylethylen stehen,
gegebenenfalls in Gegenwart eines Säurebindemittels und gegebenenfalls in Gegenwart eines Verdünnungsmittels umsetzt,
oder
c) Carboxamid-Boronsäure-Derivate der Formel (VI) 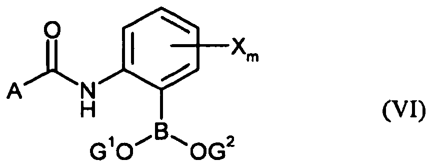 in welcher
A, X und m die in Anspruch 1 angegebenen Bedeutungen haben und
G¹ und G² jeweils für Wasserstoff oder zusammen für Tetramethylethylen stehen,
mit Phenyloxim-Derivaten der Formel (VII) 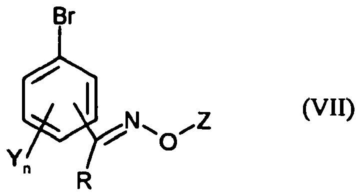 in welcher
R, Z, Y und n die in Anspruch 1 angegebenen Bedeutungen haben,
gegebenenfalls in Gegenwart eines Säurebindemittels und gegebenenfalls in Gegenwart eines Verdünnungsmittels umsetzt,
oder
d) Biphenylacyl-Derivate der Formel (VIII) 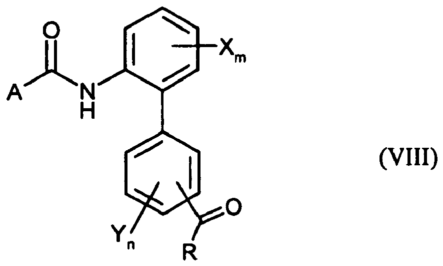 in welcher
A, R, X, Y, m und n die in Anspruch 1 angegebenen Bedeutungen haben,
mit Alkoxaminen der Formel (IX)
Z-O-NH₂ x HCl (IX)
in welcher Z die in Anspruch 1 angegebenen Bedeutungen hat,
gegebenenfalls in Gegenwart eines Säurebindemittels und gegebenenfalls in Gegenwart eines Verdünnungsmittels umsetzt,
oder
e) Hydroxylamin-Derivate der Formel (I-a) 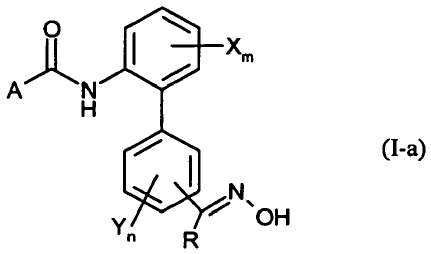 in welcher
A, R, X, Y, m und n die in Anspruch 1 angegebenen Bedeutungen haben,
mit Verbindungen der Formel (X) 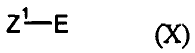 in welcher
Z¹ für C₁-C₆-Alkyl steht und
E für Chlor, Brom, Iod, Methansulfonyl oder p-Toluolsulfonyl steht,
oder
Z¹ und E zusammen für (Di-C₁-C₆-alkyl)sulfat stehen,
gegebenenfalls in Gegenwart eines Säurebindemittels und gegebenenfalls in Gegenwart eines Verdünnungsmittels umsetzt,
oder
f) Carboxamid-Derivate der Formel (IV) 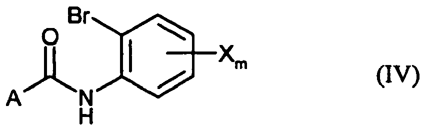 in welcher
A, X und m die in Anspruch 1 angegebenen Bedeutungen haben,
mit Phenyloxim-Derivaten der Formel (VII) 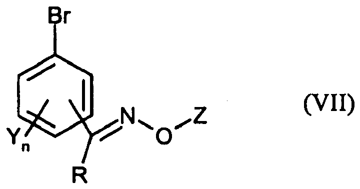 in welcher
R, Z, Y und n die in Anspruch 1 angegebenen Bedeutungen haben,
in Gegenwart eines Palladium- oder Platin-Katalysators und in Gegenwart von 4,4,4',4',5,5,5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt.

5. Anilin-Derivate der Formel (III) 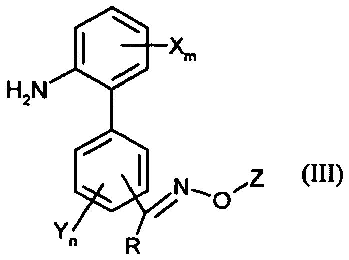 in welcher
R, Z, X, Y, m und n die in Anspruch 1 angegebenen Bedeutungen haben.

6. Verfahren zur Herstellung von Anilin-Derivaten der Formel (III) gemäß Anspruch 5, **dadurch gekennzeichnet, dass** man
g) 2-Halogenanilin-Derivate der allgemeinen Formel (XI) 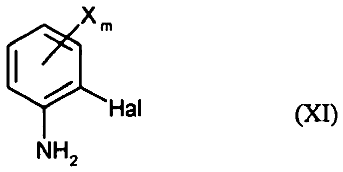 in welcher
X und m die in Anspruch 1 angegebenen Bedeutungen haben und
Hal für Halogen steht,
mit Boronsäure-Derivaten der Formel (V) 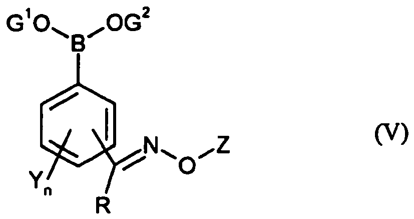 in welcher
R, Z, Y und n die in Anspruch 1 angegebenen Bedeutungen haben und
G¹ und G² jeweils für Wasserstoff oder zusammen für Tetramethylethylen stehen,
gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt
oder
h) Anilinboronsäuren der Formel (XII) 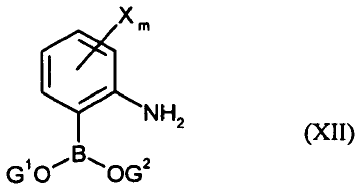 in welcher
X und m die in Anspruch 1 angegebenen Bedeutungen haben und
G¹ und G² jeweils für Wasserstoff oder zusammen für Tetramethylethylen stehen,
mit Phenyloxim-Derivaten der Formel (VII) 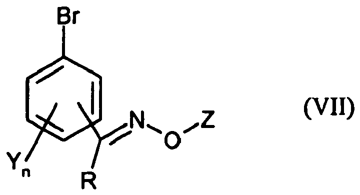 in welcher
R, Z, Y und n die in Anspruch 1 angegebenen Bedeutungen haben,
gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt.

7. Carboxamid-Boronsäure-Derivate der Formel (VI) 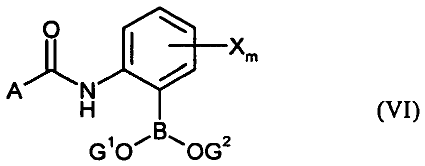 in welcher
A, X und m die in Anspruch 1 angegebenen Bedeutungen haben und
G¹ und G² jeweils für Wasserstoff oder zusammen für Tetramethylethylen stehen.

8. Verfahren zur Herstellung von Carboxamid-Boronsäure-Derivaten der Formel (VI) gemäß Anspruch 9, **dadurch gekennzeichnet, dass** man
j) Carbonsäure-Derivate der Formel (II) 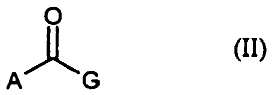 in welcher
A und G die in Anspruch 1 angegebenen Bedeutungen haben,
mit Anilinboronsäuren der Formel (XII) 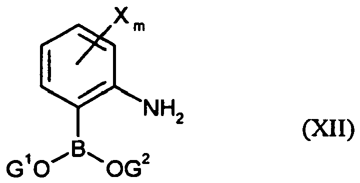 in welcher
X und m die in Anspruch 1 angegebenen Bedeutungen haben und
G¹ und G² jeweils für Wasserstoff oder zusammen für Tetramethylethylen stehen,
gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt.

9. Biphenylacyl-Derivate der Formel (VIII) 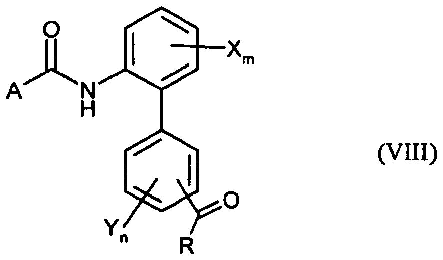 in welcher
A, R, X, Y, m und n die in Anspruch 1 angegebenen Bedeutungen haben.

10. Verfahren zur Herstellung von Biphenylacyl-Derivaten der Formel (VIII) gemäß Anspruch 11, **dadurch gekennzeichnet, dass** man
k) Carbonsäure-Derivate der Formel (II) 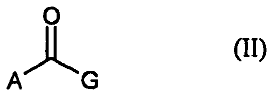 in welcher
A und G die in Anspruch 1 angegebenen Bedeutungen haben,
mit 2-Benzaldehyd-anilin-Derivaten der Formel (XIV) 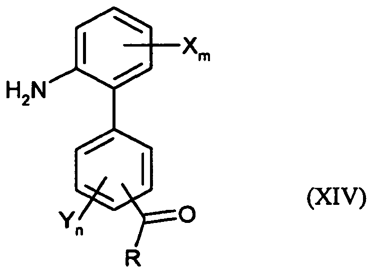 in welcher
R, X, Y, m und n die in Anspruch 1 angegebenen Bedeutungen haben,
gegebenenfalls in Gegenwart eines Säurebindemittels, sowie gegebenenfalls in Gegenwart eines inerten organischen Verdünnungsmittels umsetzt.

11. Mittel zur Bekämpfung unerwünschter Mikroorganismen, **gekennzeichnet durch** einen Gehalt an mindestens einem Biphenylcarboxamid der Formel (I) gemäß Anspruch 1 neben Streckmitteln und/oder oberflächenaktiven Stoffen.

12. Verwendung von Biphenylcarboxamiden der Formel (I) gemäß Anspruch 1 zur Bekämpfung unerwünschter Mikroorganismen, ausserhalb des menschlichen oder tierschen Körpers.

13. Verfahren zur Bekämpfung unerwünschter Mikroorganismen im Pfanzenschutz und im Materialschutz, **dadurch gekennzeichnet, dass** man Biphenylcarboxamide der Formel (I) gemäß Anspruch 1 auf die Mikroorganismen und/oder deren Lebensraum ausbringt.

14. Verfahren zur Herstellung von Mitteln zur Bekämpfung unerwünschter Mikroorganismen ausserhalb des menschlichen oder tierischen Körpers, **dadurch gekennzeichnet, dass** man Biphenylcarboxamide der Formel (I) gemäß Anspruch 1 mit Streckmitteln und/oder oberflächenaktiven Stoffen vermischt.

## Claims

1. Biphenylcaxboxamides of the formula (I) 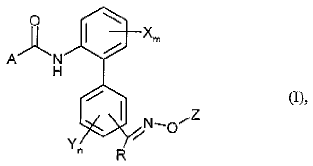 in which
R represents hydrogen, C₁-C₆-alkyl, C₃-C₆-cycloalkyl or represents C₁-C₆-halogenoalkyl having 1 to 5 halogen atoms,
Z represents hydrogen, C₁-C₆-alkyl or represents C₁-C₆-halogenoalkyl having 1 to 5 halogen atoms,
X and Y independently of one another each represent halogen, nitro, cyano, hydroxy, carboxyl, C₁-C₈-alkyl, C₁-C₆-halogenoalkyl having 1 to 5 halogen atoms, C₁-C₈-alkoxy, C₁-C₆-halogenoalkoxy having 1 to 5 halogen atoms, C₁-C₈-alkylthio, C₁-C₆-halogenoalkylthio having 1 to 5 halogen atoms, C₂-C₈-alkenyloxy, C₂-C₈-halogenoalkenyloxy having 1 to 5 halogen atoms, C₃-C₈-alkinyloxy, C₃-C₈-halogenoalkinyloxy having 1 to 5 halogen atoms, C₃-C₈-cycloalkyl, C₁-C₈-alkoxycarbonyl, C₁-C₈-alkylsulphinyl, C₁-C₈-alkylsulphonyl, C₁-C₈-halogenoalkylsulphinyl having 1 to 5 halogen atoms, C₁-C₈-halogenoalkylsulphonyl having 1 to 5 halogen atoms or C₁-C₆-alkoximino-C₁-C₆-alkyl,
m represents integers from 0 to 3, where X represents identical or different radicals ifm represents 2 or 3,
n represents integers from 0 to 4, where Y represents identical or different radicals, if n represents 2, 3 or 4,
and
A represents a radical of the formula 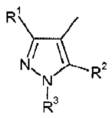 in which
α)
R¹ represents hydrogen, cyano, halogen, nitro, C₁-C₄-alkyl, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, C₁-C₄-alkylthio, C₁-C₄-halogenoalkylthio having 1 to 5 halogen atoms, aminocarbonyl, or aminocarbonyl-C₁-C₄-alkyl and
R² represents hydrogen, cyano, C₁-C₄-alkyl, C₁-C₄-alkoxy or C₁-C₄-alkylthio and
R³ represents hydrogen, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl, C₁-C₄-halogenoalkoxy-C₁-C₄-alkyl having 1 to 5 halogen atoms or phenyl,
or
β)
R¹ represents hydrogen, cyano, halogen, nitro, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, C₁-C₄-alkylthio, C₁-C₄-halogenoalkylthio having 1 to 5 halogen atoms, aminocarbonyl, or aminocarbonyl-C₁-C₄-alkyl and
R² represents fluorine and
R³ represents hydrogen, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl, C₁-C₄-halogenoalkoxy-C₁-C₄-alkyl having 1 to 5 halogen atoms or phenyl,
or
γ)
R¹ represents hydrogen, cyano, halogen, nitro, C₁-C₄-alkyl, C₁-C₄-halogenoalkyl having 1 to 5 halogen atoms, C₃- C₆-cycloalkyl, C₁-C₄-alkoxy, C₁-C₄-halogenoalkoxy having 1 to 5 halogen atoms, C₁-C₄-alkylthio, C₁-C₄-halogenoalkylthio having I to 5 halogen atoms, aminocarbonyl, or aminocarbonyl-C₁-C₄-alkyl and
R² represents fluorine and
R³ represents hydrogen, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl, C₁-C₄-halogenoalkoxy-C₁-C₄-alkyl having I to 5 halogen atoms or phenyl.

2. Biphenylcarboxamides of the formula (I) according to Claim 1 in which
R represents hydrogen, C₁-C₄-alkyl, C₃-C₆-cycloalkyl or C₁-C₄-halogenoalkyl having 1 to 5 halogen atoms,
Z represents hydrogen, C₁-C₄-alkyl or C₁-C₄-halogenoalkyl having 1 to 5 halogen atoms,
X and Y independently of one another each represent fluorine, chloride, bromine, nitro, cyano, hydroxy, carboxyl, C₁-C₆-alkyl, C₁-C₂-halogenoalkyl having 1 to 5 fluorine, chlorine and/or bromine atoms, C₁-C₆-alkoxy, C₁-C₂-halogenoalkoxy having 1 to 5 fluorine, chlorine and/or bromine atoms, C₁-C₆-alkylthio, C₁-C₂-halogenoalkylthio having I to 5 fluorine, chlorine and/or bromine atoms, C₂-C₆-alkenyloxy, C₂-C₆-halogenoalkenyloxy having 1 to 5 halogen atoms, C₃-C₆-alkinyloxy, C₃-C₆-halogenoalkinyloxy having 1 to 5 halogen atoms, C₃-C₇-cycloalkyl, C₁-C₄-alkoxycarbonyl, C₁-C₆-alkylsulphinyl, C₁-C₆-alkylsulphonyl, C₁-C₆-halogenoalkylsulphinyl having 1 to 5 halogen atoms, C₁-C₆-halogenoalkylsulphoyl having 1 to 5 halogen atoms or represents C₁-C₄-alkoximino-C₁-C₄-alkyl,
m represents integers from 0 to 3, where X represents identical or different radicals if m represents 2 or 3,
n represents integers from 0 to 4, where Y represents identical or different radicals if n represents 2, 3 or 4,
and
A represents a radical of the formula 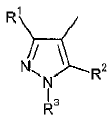 in which
α)
R¹ represents hydrogen, cyano, fluorine, chlorine, bromine, iodine, methyl, ethyl, isopropyl, C₁-C₂-halogenoalkyl having 1 to 5 fluorine, chlorine and/or bromine atoms, cyclopropyl, methoxy, ethoxy, C₁-C₂-halogenoalkoxy having 1 to 5 fluorine, chlorine and/or bromine atoms, methylthio, ethylthio, trifluoromethylthio, difluoromethylthio, aminocarbonyl, aminocarbonylmethyl or aminocarbonylethyl,
R² represents hydrogen, methyl ethyl, methoxy, ethoxy, methylthio or ethylthio and
R³ represents hydrogen, methyl, ethyl, n-propyl, isopropyl, C₁-C₂-halogenoalkyl having 1 to 5 fluorine, chlorine and/or bromine atoms, hydroxymethyl, hydroxyethyl cyclopropyl, cyclopentyl, cyclohexyl or phenyl,
or
β)
R¹ represents hydrogen, cyano, fluorine, chlorine, bromine, iodine, C₁-C₂-halogenoalkyl having 1 to 5 fluorine, chlorine and/or bromine atoms, cyclopropyl, methoxy, ethoxy, C₁-C₂-halogenoalkoxy having 1 to 5 fluorine, chlorine and/or bromine atoms, methylthio, ethylthio, trifluoromethylthio, difluoromethylthio, aminocarbonyl, aminocarbonylmethyl or aminocarbonylethyl,
R² represents fluorine and
R³ represents hydrogen, methyl, ethyl, n-propyl, isopropyl, C₁-C₂-halogenoalkyl having 1 to 5 fluorine, chlorine and/or bromine atoms, hydroxymethyl, hydroxyethyl, cyclopropyl, cyclopentyl, cyclohexyl or phenyl,
or
γ)
R¹ represents hydrogen, cyano, fluorine, chlorine, bromine, iodine, methyl, ethyl, isopropyl, C₁-C₂-halogenoalkyl having 1 to 5 fluorine, chlorine and/or bromine atoms, cyclopropyl, methoxy, ethoxy, C₁-C₂-halogenoalkoxy having 1 to 5 fluorine, chlorine and/or bromine atoms, methylthio, ethylthio, trifluoromethylthio, difluoromethylthio, aminocarbonyl, aminocarbonylmethyl or aminocarbonylethyl,
R² represents fluorine and
R³ represents hydrogen, C₁-C₂-halogenoalkyl having 1 to 5 fluorine, chlorine and/or bromine atoms, hydroxymethyl, hydroxyethyl, cyclopropyl, cyclopentyl, cyclohexyl or phenyl.

3. Biphenylcarboxamides of the formula (I) according to Claim 1 in which
R represents hydrogen, C₁-C₄-alkyl,
Z represents hydrogen, C₁-C₄-alkyl,
X and Y independently of one another each represent fluorine, chlorine, bromine, nitro, cyano, hydroxy, carboxyl, methyl, ethyl, n-propyl, isopropyl, n-butyl, sec-butyl, i-butyl, tert-butyl, trichloromethyl, trifluoromethyl, difluoromethyl, difluorochloromethyl, methoxy, ethoxy, difluoromethoxy, trifluoromethoxy, methylthio, trifluoromethylthio, difluorochloromethylthio, allyloxy, propargyloxy, cyclopropyl, cyclopentyl, cyclohexyl, cycloheptyl, methoxycarbonyl, ethoxycarbonyl, methoximinomethyl, ethoximinomethyl, methoximinoethyl or ethoximinoethyl,
m represents integers from 0 to 3, where X represents identical or different radicals if m represents 2 or 3,
n represents the numbers 0 to 4, where Y represents identical or different radicals if n represents 2, 3 or 4,
and
A represents a radical of the formula 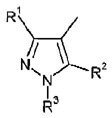 in which
α)
R¹ represents hydrogen, fluorine, chlorine, bromine, iodine, methyl, ethyl, isopropyl, monofluoromethyl, difluoromethyl, trifluoromethyl, difluorochloromethyl, trichloromethyl, cyclopropyl, methoxy, ethoxy, trifluoromethoxy, trichloromethoxy, methylthio, ethylthio, trifluoromethylthio or difluoromethylthio and
R² represents hydrogen, methyl, ethyl, methoxy, ethoxy, methylthio or ethylthio and
R³ represents hydrogen, methyl, ethyl, trifluoromethyl, difluoromethyl, hydroxymethyl, hydroxyethyl or phenyl,
or
β)
R¹ represents hydrogen, fluorine, chlorine, bromine, iodine, monofluoromethyl, difluoromethyl, trifluoromethyl, difluorochloromethyl, trichloromethyl, cyclopropyl, methoxy, ethoxy, trifluoromethoxy, trichloromethoxy, methylthio, ethylthio, trifluoromethylthio or difluoromethylthio and
R² represents fluorine and
R³ represents hydrogen, methyl, ethyl, trifluoromethyl, difluoromethyl, hydroxymethyl, hydroxyethyl or phenyl,
or
γ)
R¹ represents hydrogen, fluorine, chlorine, bromine, iodine, methyl, ethyl, isopropyl, monofluoromethyl, difluoromethyl, trifluoromethyl, difluorochloromethyl, trichloromethyl, cyclopropyl, methoxy, ethoxy, trifluoromethoxy, trichloromethoxy, methylthio, ethylthio, trifluoromethylthio or difluoromethylthio and
R² represents fluorine and
R³ represents hydrogen, trifluoromethyl, difluoromethyl, hydroxymethyl, hydroxyethyl or phenyl.

4. Process for preparing biphenylcarboxamides of the formula (I) according to Claim 1, **characterized in that**
a) carboxylic acid derivatives of the formula (II) 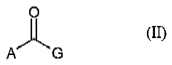 in which
A is as defined in Claim 1 and
G represents halogen, hydroxy or C₁-C₆-alkoxy,
are reacted with aniline derivatives of the formula (III) 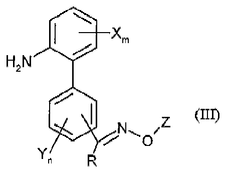 in which
R, Z, X, Y, m and n are each as defined in Claim 1,
if appropriate in the presence of a catalyst, if appropriate in the presence of an acid binder and if appropriate in the presence of a diluent,
or
b) carboxamide derivatives of the formula (IV) 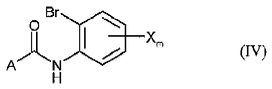 in which
A, X and m are each as defined in Claim 1,
are reacted with boronic acid derivatives of the formula (V) 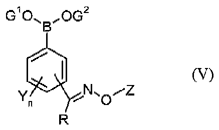 in which
R, Z, Y and n are each as defined in Claim 1 and
G¹ and G² each represent hydrogen or together represent tetramethylethylene,
if appropriate in the presence of an acid binder and if appropriate in the presence of a diluent,
or
c) carboxamide-boronic acid derivatives of the formula (VI) 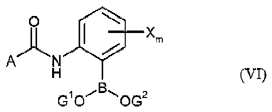 in which A, X and m are each as defined in Claim 1 and
G¹ and G² each represent hydrogen or together represent tetramethylethylene,
are reacted with phenyloxime derivatives of the formula (VII) 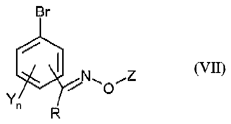 in which
R, Z, Y and n are each as defined in Claim 1,
if appropriate in the presence of an acid binder and if appropriate in the presence of a diluent,
or
d) biphenylacyl derivatives of the formula (VIII) 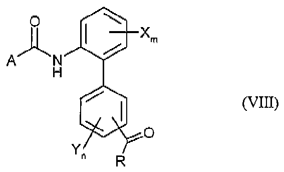 in which A, R, X, Y, m and n are each as defined in Claim 1,
are reacted with alkoxamines of the formula (IX)
Z-O-NH₂ x HCl (IX)
in which Z are each as defined in Claim 1,
if appropriate in the presence of an acid binder and if appropriate in the presence of a diluent,
or
e) hydroxyamine derivatives of the formula (I-a) 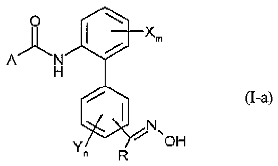 in which
A, R, X, Y, m and n are each as defined in Claim 1,
are reacted with compounds of the formula (X) 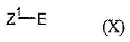 in which
Z¹ represents C₁-C₆-alkyl and
E represents chlorine, bromine, iodine, methansulphonyl or p-toluenesulphonyl,
or
Z¹ and E together represent (di-C₁-C₆-alkyl) sulphate,
if appropriate in the presence of an acid binder and if appropriate in the presence of a diluent,
or
f) carboxamide derivatives of the formula (IV) 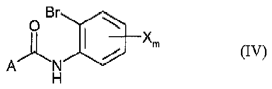 in which
A, X and m are each as defined in Claim 1,
are reacted with phenyloxime derivatives of the formula (VII) 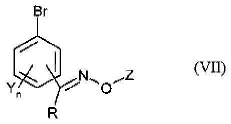 in which
R, Z, Y and n are each as defined in Claim 1,
in the presence of a palladium or platinum catalyst and in the presence of 4,4,4',4',5,5,5',5'-octamethyl-2,2'-bis-1,3,2-dioxaborolane, if appropriate in the presence of an acid binder and if appropriate in the presence of a diluent.

5. Aniline derivatives of the formula (III) 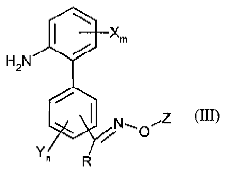 in which
R, Z, X, Y, m and n are each as defined in Claim 1.

6. Process for preparing aniline derivatives of the formula (III) according to Claim 5, **characterized in that**
g) 2-halogenoaniline derivatives of the general formula (XI) 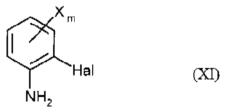 in which
X and m are each as defined in Claim 1 and
Hal represents halogen,
are reacted with boronic acid derivatives of the formula (V) 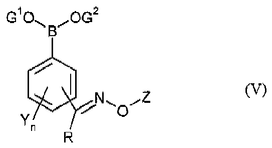 in which
R, Z, Y and n are each as defined in Claim 1 and
G¹ and G² each represent hydrogen or together represent tetramethylethylene,
if appropriate in the presence or an acid binder, and if appropriate in the presence of an inert organic diluent, and if appropriate in the presence of a catalyst
or
h) aniline boronic acids of the formula (XII) 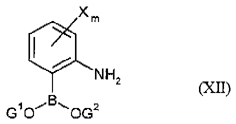 in which
X and m are each as defined in Claim 1 and
G¹ and G² each represent hydrogen or together represent tetramethytethytene,
are reacted with phenyloxime derivatives of the formula (VII) 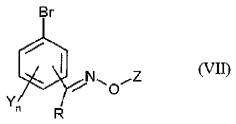 in which
R, Z, Y and n are each as defined in Claim 1,
if appropriate in the presence of an acid binder, and if appropriate in the presence of an inert organic diluent, and if appropriate in the presence of a catalyst.

7. Carboxamide-boronic acid derivatives of the formula (VI) 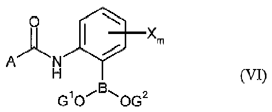 in which
A, X and m are each as defined in Claim 1 and
G¹ and G² each represent hydrogen or together represent tetramethylethylene.

8. Process for preparing carboxamide-boronic acid derivatives of the formula (VI) according to Claim 7, **characterized in that**
j) carboxylic acid derivatives of the formula (II) 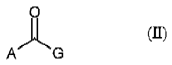 in which
A and G are each as defined in Claim 1,
are reacted with aniline boronic acids of the formula (XII) 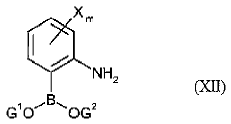 in which X and in are each as defined in Claim 1 and
G¹ and G² each represent hydrogen or together represent tetramethylethylene,
if appropriate in the presence of an acid binder, and if appropriate in the presence of an inert organic diluent, and if appropriate in the presence of a catalyst.

9. Biphenylacyl derivatives of the formula (VIII) 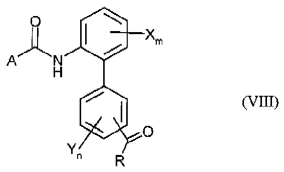 in which
A, R, X, Y, m and n are each as defined in Claim 1.

10. Process for preparing biphenylacyl derivatives of the formula (VIII) according to Claim 9, **characterised in that**
k) carboxylic acid derivatives of the formula (II) 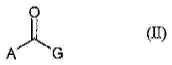 in which
A and G are each as defined in Claim 1,
are reacted with 2-benzaldelayde-aniline derivatives of the formula (XIV) 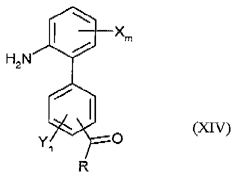 in which
R, X, Y, m and n are each as defined in Claim 1,
if appropriate in the presence of an acid binder, and if appropriate in the presence of an inert organic diluent.

11. Composition for controlling undesirable microorganisms, **characterized by** a content of at least one biphenylcarboxamide of the formula (I) according to Claim 1, in addition to extenders and/or surfactants,

12. Use of biphenylcarboxamides of the formula (I) according to Claim 1 for controlling undesirable microorganisms outside the human or animal body.

13. Method for controlling undesirable microorganisms in crop protection and in the protection of materials, **characterized in that** biphenylcarboxamides of the formula (I) according to Claim 1 are applied on to the microorganisms and/or their habitat.

14. Process for preparing compositions for controlling indesirable microorganisms outside the human or animal body, **characterized in that** biphenylcarboxamides of the formula (I) according to Claim 1 are mixed with extenders and/or surfactants.

## Revendications

1. Biphénylcarboxamides de formule (I) 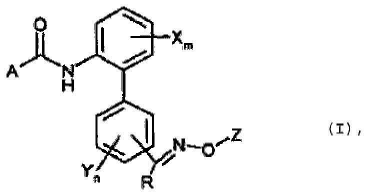 dans laquelle
R représente un atome d'hydrogène, un groupe alkyle en C₁-C₆, cycloalkyle en C₃-C₆ ou un groupe halogénoalkyle en C₁-C₆ comportant de 1 à 5 atomes d'halogène,
Z représente un atome d'hydrogène, un groupe alkyle en C₁-C₆ ou un groupe halogénoalkyle en C₁-C₆ comportant de 1 à 5 atomes d'halogène,
X et Y représentent indépendamment l'un de l'autre un atome d'halogène, un groupe nitro, cyano, hydroxy, carboxy, alkyle en C₁-C₈, halogénoalkyle en C₁-C₆ comportant de 1 à 5 atomes d'halogène, alcoxy en C₁-C₈, halogénoalcoxy en C₁-C₆ comportant de 1 à 5 atomes d'halogène, alkyl(C₁-C₆)thio, halogénoalkyl(C₁-C₆)thio comportant de 1 à 5 atomes d'halogène, alcényloxy en C₂-C₈, halogénoalcényloxy en C₂-C₈ comportent de 1 à atomes d'halogène, alcynyloxy en C₃-C₈, halogénoalcynyloxy en C₃-C₈ comportant de 1 à 5 atomes d'halogène, cycloalkyle en C₃-C₈, alcoxy(C₁-C₈)carbonyle, alkyl (C₁-C₈)sulfinyle, alkyl(C₁-C₈)sulfonyle, halogénoalkyl(C₁-C₈)sulfinyle comportant de 1 à 5 atomes d'halogène, halogénoalkyl (C₁-C₈) sulfonyle comportant de 1 à 5 atomes d'halogène ou alcoxy(C₁-C₆) imino-alkyle (C₁-C₆) ,
m représente des nombres entiers valant de 0 à 3, X représentant des radicaux identiques ou différents lorsque m représente 2 ou 3,
n représente des nombres entiers valant de 0 à 4, Y représentant des radicaux identiques ou différents lorsque n représente 2, 3 ou 4,
et
A représente un radical de formule 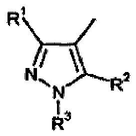 dans laquelle
α)
R¹ représente un atome d'hydrogène ou d'halogène, un groupe cyano, nitro, alkyle en C₁-C₄, halogénoalkyle en C₁-C₄ comportant de 1 à 5 atomes d'halogène, cycloalkyle en C₃-C₆, alcoxy en C₁-C₄, halogénoalcoxy en C₁-C₄ comportant de 1 à 5 atomes d'halogène, alkyl (C₁-C₄) thio, halogénoalkyl (C₁-C₄) thio comportant de 1 à 5 atomes d'halogène, aminocarbonyle, ou aminocarbonylalkyle(C₁-C₄) et
R² représente un atome d'hydrogène, un groupe cyano, alkyle en C₁-C₄, alcoxy en C₁-C₄ ou alkyl(C₁-C₄)thio et
R³ représente un atome d'hydrogène, un groupe alkyle en C₁-C₄, halogénoalkyle en C₁-C₄ comportant de 1 à 5 atomes d'halogène, hydroxyalkyle en C₁-C₄, alcényle en C₂-C₆, cycloalkyle en C₃-C₆, alkyl (C₁-C₄) thio-alkyle(C₁-C₄) , halogénoalkyl (C₁-C₄) thio-alkyle(C₁-C₄) comportant de 1 à 5 atomes d'halogène, alcoxy (C₁-C₄)-alkyle (C₁-C₄), halogénoalcoxy(C₁-C₄) -alkyle (C₁-C₄) comportant de 1 à 5 atomes d'halogène ou phényle,
ou
β)
R¹ représente un atome d'hydrogène ou d'halogène ou un groupe cyano, nitro, halogénoalkyle en C₁-C₄ comportant de 1 à 5 atomes d'halogène, cycloalkyle en C₃-C₆, alcoxy en C₁-C₄, halogénoalcoxy en C₁-C₄ comportant de 1 à 5 atomes d'halogène, alkyl (C₁-C₄) thio, halogénoalkyl (C₁-C₄) thio comportant de 1 à 5 atomes d'halogène, aminocarbonyle, ou aminocarbonylalkyle(C₁-C₄) et
R² représente un atome de fluor et
R³ représente un atome d'hydrogène, un groupe alkyle en C₁-C₄, halogénoalkyle en C₁-C₄ comportant de 1 à 5 atomes d'halogène, hydroxyalkyle en C₁-C₄, alcényle en C₂-C₆, cycloalkyle en C₃-C₆, alkyl(C₁-C₄) thio-alkyle(C₁-C₄) , halogénoalkyl(C₁-C₄)thio-alkyle(C₁-C₄) comportant de 1 à 5 atomes d'halogène, alcoxy(C₁-C₄)-alkyle(C₁-C₄), halogénoalcoxy(C₁-C₄)-alkyle(C₁-C₄) comportant de 1 à 5 atomes d'halogène ou phényle,
ou
γ)
R¹ représente un atome d'hydrogène ou d'halogène ou un groupe cyano, nitro, alkyle en C₁-C₄, halogénoalkyle en C₁-C₄ comportant de 1 à 5 atomes d'halogène, cycloalkyle en C₃-C₆, alcoxy en C₁-C₄, halogénoalcoxy en C₁-C₄ comportant de 1 à 5 atomes d'halogène, alkyl(C₁-C₄) thio, halogénoalkyl (C₁-C₄) thio comportant de 1 à 5 atomes d'halogène, aminocarbonyle, ou aminocarbonyl-alkyle(C₁-C₄) et
R² représente un atome de fluor et
R³ représente un atome d'hydrogène, un groupe halogénoalkyle en C₁-C₄ comportant de 1 à 5 atomes d'halogène, hydroxyalkyle en C₁-C₄, alcényle en C₂-C₆, cycloalkyle en C₃-C₆, alkyl (C₁-C₄) thio-alkyle (C₁-C₄) , halogénoalkyl(C₁-C₄)thio-alkyle(C₁-C₄) comportant de 1 à 5 atomes d'halogène, alcoxy (C₁-C₄)-alkyle (C₁-C₄) , halogénoalcoxy (C₁-C₄)-alkyle (C₁-C₄) comportant de 1 à 5 atomes d'halogène ou phényle.

2. Biphénylcarboxamides de formule (I) selon la revendication 1, dans lesquels
R représente un atome d'hydrogène, un groupe alkyle en C₁-C₄, cycloalkyle en C₃-C₆ ou un groupe halogénoalkyle en C₁-C₄ comportant de 1 à 5 atomes d'halogène,
Z représente un atome d'hydrogène, un groupe alkyle en C₁-C₄ ou un groupe halogénoalkyle en C₁-C₄ comportant de 1 à 5 atomes d'halogène,
X et Y représentent indépendamment l'un de l'autre un atome de fluor, chlore, brome, un groupe nitro, cyano, hydroxy, carboxy, alkyle en C₁-C₆, halogénoalkyle en C₁-C₂ comportant de 1 à 5 atomes de fluor, chlore et/ou brome, alcoxy en C₁-C₆, halogénoalcoxy en C₁-C₂ comportant de 1 à 5 atomes de fluor, chlore et/ou brome, alkyl(C₁-C₆)thio, halogénoalkyl(C₁-C₂)thio comportant de 1 à 5 atomes de fluor, chlore et/ou brome, alcényloxy en C₂-C₆, halogénoalcényloxy en C₂-C₆ comportant de 1 à 5 atomes d'halogène, alcynyloxy en C₃-C₆, halogénoalcynyloxy en C₃-C₆ comportant de 1 à 5 atomes d'halogène, cycloalkyle en C₃-C₇, alcoxy(C₁-C₄)-carbonyle, alkyl(C₁-C₆)sulfinyle, alkyl(C₂-C₆)-sulfonyle, halogénoalkyl(C₁-C₆)sulfinyle comportant de 1 à 5 atomes d'halogène, halogénoalkyl(C₁-C₆)-sulfonyle comportant de 1 à 5 atomes d'halogène ou alcoxy(C₁-C₄) imino-alkyle(C₁-C₄) ,
m représente des nombres entiers valant de 0 à 3, X représentant des radicaux identiques ou différents lorsque m représente 2 ou 3,
n représente des nombres entiers valant de 0 à 4, Y représentant des radicaux identiques ou différents lorsque n représente 2, 3 ou 4,
et
A représente un radical de formule 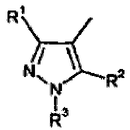 dans laquelle
α)
R¹ représente un atome d'hydrogène, de fluor, chlore, brome, iode, un groupe cyano, méthyle, éthyle, isopropyle, halogénoalkyle en C₁-C₂ comportant de 1 à 5 atomes de fluor, chlore et/ou brome, cyclopropyle, méthoxy, éthoxy, halogénoalcoxy en C₁-C₂ comportant de 1 à 5 atomes de fluor, chlore et/ou brome, méthylthio, éthylthio, trifluorométhylthio, difluorométhylthio, aminocarbonyle, aminocarbonylméthyle ou aminocarbonyléthyle,
R² représente un atome d'hydrogène, un groupe méthyle, éthyle, méthoxy, éthoxy, méthylthio ou éthylthio et
R³ représente un atome d'hydrogène, un groupe méthyle, éthyle, n-propyle, isopropyle, halagénoalkyle en C₁-C₂ comportant de 1 à 5 atomes de fluor, chlore et/ou brome, hydroxyméthyle, hydroxyéthyle, cyclopropyle, cyclopentyle, cyclohexyle ou phényle,
ou
β)
R¹ représente un atome d'hydrogène, de fluor, chlore, brome, iode, un groupe cyano, halogénoalkyle en C₁-C₂ comportant de 1 à 5 atomes de fluor, chlore et/ou brome, cyclopropyle, méthoxy, éthoxy, halogénoalcoxy en C₁-C₂ comportant de 1 à 5 atomes de fluor, chlore et/ou brome, méthylthio, éthylthio, trifluorométhylthio, difluorométhylthio, aminocarbonyle, aminocarbonylméthyle ou aminocarbonyléthyle,
R² représente un atome de fluor et
R³ représente un atome d'hydrogène, un groupe méthyle, éthyle, n-propyle, isopropyle, halogénoalkyle en C₁-C₂ comportant de 1 à 5 atomes de fluor, chlore et/ou brome, hydroxyméthyle, hydroxyéthyle, cyclopropyle, cyclopentyle, cyclohexyle ou phényle,
ou
γ)
R¹ représente un atome d'hydrogène, de fluor, chlore, brome, iode, un groupe cyano, méthyle, éthyle, isopropyle, halogénoalkyle en C₁-C₂ comportant de 1 à 5 atomes de fluor, chlore et/ou brome, cyclopropyle, méthoxy, éthoxy, halogénoalcoxy en C₁-C₂ comportant de 1 à 5 atomes de fluor, chlore et/ou brome, méthylthio, éthylthio, trifluorométhylthio, difluorométhylthio, aminocarbonyle, aminocarbonylméthyle ou aminocarbonyléthyle,
R² représente un atome de fluor et
R³ représente un atome d'hydrogène, un groupe halogénoalkyle en C₁-C₂ comportant de 1 à 5 atomes de fluor, chlore et/ou brome, hydroxyméthyle, hydroxyéthyle, cyclopropyle, cyclopentyle, cyclohexyle ou phényle.

3. Biphénylcarboxamides de formule (I) selon la revendication 1, dans lesquels
R représente un atome d'hydrogène, un groupe alkyle en C₁-C₄,
Z représente un atome d'hydrogène, un groupe alkyle en C₁-C₄,
X et Y représentent indépendamment l'un de l'autre un atome de fluor, chlore, brome, un groupe nitro, cyano, hydroxy, carboxy, méthyle, éthyle, n-propyle, isopropyle, n-butyle, sec-butyle, isobutyle, tert-butyle, trichlorométhyle, trifluorométhyle, difluorométhyle, difluorochlorométhyle, méthoxy, éthoxy, difluorométhoxy, trifluorométhoxy, méthylthio, trifluorométhylthio, difluorochlorométhylthio, allyloxy, propargyloxy, cyclopropyle, cyclopentyle, cyclohexyle, cycloheptyle, méthoxycarbonyle, éthoxycarbonyle, méthoxyiminométhyle, éthoxyiminométhyle, méthoxyiminoéthyle ou éthoxyiminoéthyle,
m représente des nombres entiers valant de 0 à 3, X représentant des radicaux identiques ou différents lorsque m représente 2 ou 3,
n représente des nombres entiers valant de 0 à 4, Y représentant des radicaux identiques ou différents lorsque n représente 2, 3 ou 4,
et
A représente un radical de formule 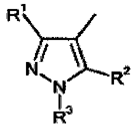 dans laquelle
α)
R¹ représente un atome d'hydrogène, de fluor, chlore, brome, iode, un groupe méthyle, éthyle, isopropyle, monofluorométhyle, difluorométhyle, trifluorométhyle, difluorochlorométhyle, trichlorométhyle, cyclopropyle, méthoxy, éthoxy, trifluorométhoxy, trichlorométhoxy, méthylthio, éthylthio, trifluorométhylthio ou difluorométhylthio et
R² représente un atome d'hydrogène, un groupe méthyle, éthyle, méthoxy, éthoxy, méthylthio ou éthylthio et
R³ représente un atome d'hydrogène, un groupe méthyle, éthyle, trifluorométhyle, difluorométhyle, hydroxyméthyle, hydroxyéthyle ou phényle,
ou
β)
R¹ représente un atome d'hydrogène, de fluor, chlore, brome, iode, un groupe monofluorométhyle, difluorométhyle, trifluorométhyle, difluorochlorométhyle, trichlorométhyle, cyclopropyle, méthoxy, éthoxy, trifluorométhoxy, trichlorométhoxy, méthylthio, éthylthio, trifluorométhylthio ou difluorométhylthio et
R² représente un atome de fluor et
R³ représente un atome d'hydrogène, un groupe méthyle, éthyle, trifluorométhyle, difluorométhyle, hydroxyméthyle, hydroxyéthyle ou phényle,
ou
γ)
R¹ représente un atome d'hydrogène, de fluor, chlore, brome, iode, un groupe méthyle, éthyle, isopropyle, monofluorométhyle, difluorométhyle, trifluorométhyle, difluorochlorométhyle, trichlorométhyle, cyclopropyle, méthoxy, éthoxy, trifluorométhoxy, trichlorométhoxy, méthylthio, éthylthio, trifluorométhylthio ou difluorométhylthio et
R² représente un atome de fluor et
R³ représente un atome d'hydrogène, un groupe trifluorométhyle, difluorométhyle, hydroxyméthyle, hydroxyéthyle ou phényle.

4. Procédé pour la préparation de biphénylcarboxamides de formule (I) selon la revendication 1, **caractérisé en ce que**
a) on fait réagir des dérivées d'acides carboxyliques de formule (II) 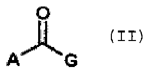 dans laquelle
A a les significations indiquées dans la revendication 1 et
G représente un atome d'halogène, un groupe hydroxy ou alcoxy en C₁-C₆, avec des dérivés d'aniline de formule (III) 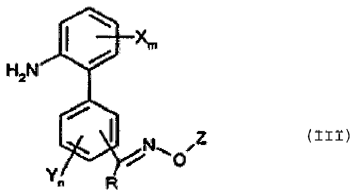 dans laquelle
R, Z, X, Y, m et n ont les significations indiquées dans la revendication 1,
éventuellement en présence d'un catalyseur, éventuellement en présence d'un capteur d'acide et éventuellement en présence d'un diluant,
ou
b) on fait réagir des dérivés de carboxamides de formule (IV) 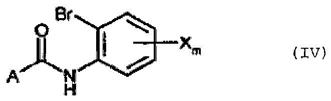 dans laquelle
A, X et m ont les significations indiquées dans la revendication 1,
avec des dérivés d'acide boronique de formule (V) 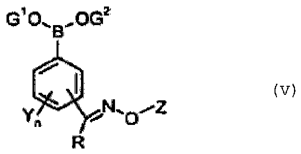 dans laquelle
R, Z, Y et n ont les significations indiquées dans la revendication 1 et
G¹ et G² représentent chacun un atome d'hydrogène ou ensemble le groupe tétraméthyléthylène,
éventuellement en présence d'un capteur d'acide et éventuellement en présence d'un diluant,
ou
c) on fait réagir des dérivés d'acide carboxamide-boronique de formule (VI) 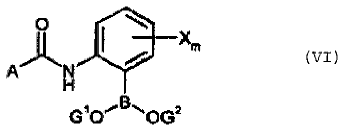 dans laquelle
A, X et m ont les significations indiquées dans la revendication 1 et
G¹ et G² représentent chacun un atome d'hydrogène ou ensemble le groupe tétraméthyléthylène,
avec des dérivés de phényloxime de formule (VII) 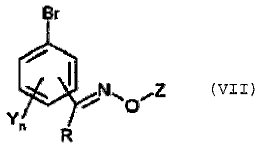 dans laquelle
R, Z, Y et n ont les significations indiquées dans la revendication 1,
éventuellement en présence d'un capteur d'acide et éventuellement en présence d'un diluant,
ou
d) on fait réagir des dérivés biphénylacyle de formule (VIII) 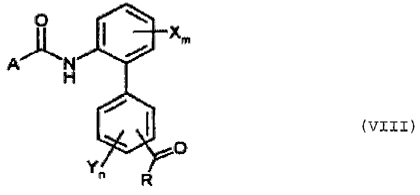 dans laquelle
A, R, X, Y, m et n ont les significations indiquées dans la revendication 1,
avec des alcoxamines de formule (IX)
Z-O-NH₂ x HCl (IX)
dans laquelle Z a les significations indiquées dans la revendication 1,
éventuellement en présence d'un capteur d'acide et éventuellement en présence d'un diluant,
ou
e) on fait réagir des dérivés d'hydroxylamine de formule (I-a) 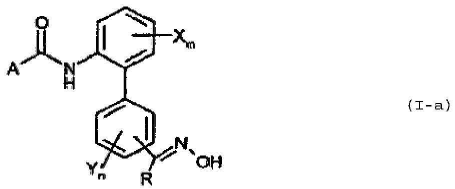 dans laquelle
A, R, X, Y, m et n ont les significations indiquées dans la revendication 1,
avec des composés de formule (X)
Z¹-E (X)
dans laquelle
Z¹ représente un groupe alkyle en C₁-C₆ et
E représente un atome de chlore, brome, iode, un groupe méthanesulfonyle ou p-toluènesulfonyle,
ou
Z¹ et E représentent ensemble un [dialkyl(C₁-C₆)]-sulfate,
éventuellement en présence d'un capteur d'acide et éventuellement en présence d'un diluant,
ou
f) on fait réagir des dérivés de carboxamides de formule (IV) 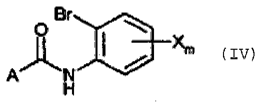 dans laquelle
A, X et m ont les significations indiquées dans la revendication 1,
avec des dérivés de phényloxime de formule (VII) 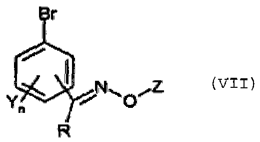 dans laquelle
R, Z, Y et n ont les significations indiquées dans la revendication 1,
en présence d'un catalyseur au palladium ou au platine et en présence de 4, 4, 4' ,4' ,5 ,5 ,5' ,5'-octaméthyl-2,2'-bis-1,3,2-dioxoborolane, éventuellement en présence d'un capteur d'acide et éventuellement en présence d'un diluant.

5. Dérivés d'aniline de formule (III) 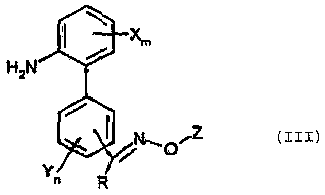 dans laquelle
R, Z, X, Y, m et n ont les significations indiquées dans la revendication 1.

6. Procédé pour la préparation de dérivés d'aniline de formule (III) selon la revendication 5, **caractérisé en ce que**
g) on fait réagir des dérivés de 2-halogénoanilines de formule générale (XI) 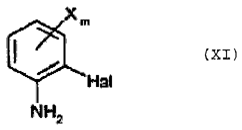 dans laquelle
X et m ont les significations indiquées dans la revendication 1 et
Hal représente un atome d'halogène,
avec des dérivés d'acide boronique de formule (V) 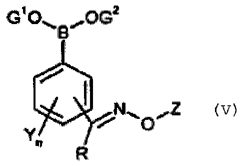 dans laquelle
R, Z, Y et n ont les significations indiquées dans la revendication 1 et
G¹ et G² représentent chacun un atome d'hydrogène ou ensemble le groupe tétraméthyléthylène,
éventuellement en présence d'un capteur d'acide, ainsi qu'éventuellement en présence d'un diluant organique inerte, ainsi qu'éventuellement en présence d'un catalyseur
ou
h) on fait réagir des acides anilineboroniques de formule (XII) 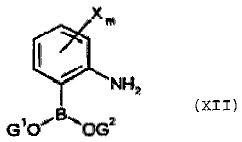 dans laquelle
X et m ont les significations indiquées dans la revendication 1 et
G¹ et G² représentent chacun un atome d'hydrogène ou ensemble le groupe tétraméthyléthylène, avec des dérivés de phényloxime de formule (VII) 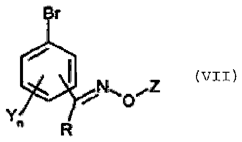 dans laquelle
R, Z, Y et n ont les significations indiquées dans la revendication 1,
éventuellement en présence d'un capteur d'acide, ainsi qu'éventuellement en présence d'un diluant organique inerte, ainsi qu'éventuellement en présence d'un catalyseur.

7. Dérivés d'acide carboxamide-boronique de formule (VI) 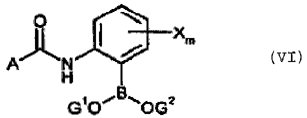 dans laquelle
A, X et m ont les significations indiquées dans la revendication 1 et
G¹ et G² représentent chacun un atome d'hydrogène ou ensemble le groupe tétraméthyléthylène.

8. Procédé pour la préparation de dérivés d'acide carboxamide-boronique de formule (VI) selon la revendication 7, **caractérisé en ce que**
j) on fait réagir des dérivés d'acides carboxyliques de formule (II) 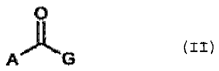 dans laquelle
A et G ont les significations indiquées dans la revendication 1,
avec des acides anilineboroniques de formule (XII) 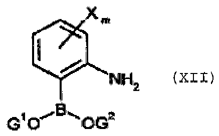 dans laquelle
X et m ont les significations indiquées dans la revendication 1 et
G¹ et G² représentent chacun un atome d'hydrogène ou ensemble le groupe tétraméthyléthylène, éventuellement en présence d'un capteur d'acide, ainsi qu'éventuellement en présence d'un diluant organique inerte, ainsi qu'éventuellement en présence d'un catalyseur.

9. Dérivés biphénylacyle de formule (VIII) 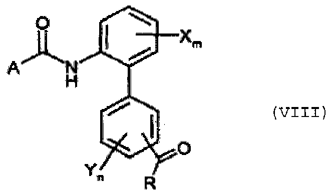 dans laquelle
A, R, X, Y, m et n ont les significations indiquées dans la revendication 1.

10. Procédé pour la préparation de dérivés biphénylacyle de formule (VIII) selon la revendication 9, **caractérisé en ce que**
k) on fait réagir des dérivés d'acides carboxyliques de formule (II) 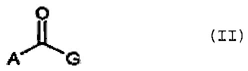 dans laquelle
A et G ont les significations indiquées dans la revendication 1,
avec des dérivés de 2-benzaldéhyde-aniline de formule (XIV) 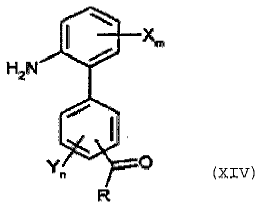 dans laquelle
R, X, Y, m et n ont les significations indiquées dans la revendication 1,
éventuellement en présence d'un capteur d'acide, ainsi qu'éventuellement en présence d'un diluant organique inerte.

11. Composition destinée à la lutte contre des micro-organismes indésirables, **caractérisée par** une teneur en au moins un biphénylcarboxamide de formule (I) selon la revendication 1, en plus d'excipients et/ou de substances tensioactives.

12. Utilisation de biphénylcarboxamides de formule (I) selon la revendication 1, pour la lutte contre des micro-organismes indésirables, hormis ceux du corps humain ou animal.

13. Procédé pour la lutte contre des micro-organismes indésirables dans la protection des plantes et dans la protection des matériaux, **caractérisé en ce qu'**on applique sur les micro-organismes et/ou leur habitat des biphénylcarboxamides de formule (I) selon la revendication 1.

14. Procédé pour la préparation de compositions destinées à la lutte contre des micro-organismes indésirables hormis ceux du corps humain ou animal, **caractérisé en ce qu'**on mélange des biphénylcarboxamides de formule (I) selon la revendication 1 avec des excipients et/ou des substances tensioactives.
